(19) **Europäisches Patentamt / European Patent Office / Office européen des brevets**

(11) **EP 4 163 391 A1**

(12) **EUROPEAN PATENT APPLICATION**

(43) Date of publication:
**12.04.2023 Bulletin 2023/15**

(21) Application number: **22199525.1**

(22) Date of filing: **04.10.2022**

(51) International Patent Classification (IPC):
**C12Q 1/6809** *(2018.01)*

(52) Cooperative Patent Classification (CPC):
(C-Sets available)
**C12Q 1/6809** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.10.2021 US 202163252818 P**
**11.05.2022 US 202263340634 P**
**20.09.2022 US 202217948837**

(71) Applicant: **JOHNSON & JOHNSON CONSUMER INC.**
**Skillman, NJ 08558 (US)**

(72) Inventors:
- **FOURRE, Tara**
**Skillman, 08558 (US)**
- **MIN, Kyungrok**
**Skillman, 08558 (US)**

(74) Representative: **Carpmaels & Ransford LLP**
**One Southampton Row**
**London WC1B 5HA (GB)**

(54) **METHOD OF QUANTIFYING PRODUCT IMPACT ON HUMAN MICROBIOME**

(57) The present invention relates to methods and kits for providing high throughput quantitative analysis of impact (e.g., by application of materials which affect - positively and/or negatively - microbial species) on human microbiome.

EP 4 163 391 A1

(52) Cooperative Patent Classification (CPC): (Cont.)

C-Sets
**C12Q 1/6809, C12Q 2535/122, C12Q 2537/165**

**Description**

CROSS-REFERENCE TO RELATED APPLICATION

The present application claims the benefit of the earlier filing date of United States provisional patent application 63/340,634, filed May 11, 2022 and United States Provisional patent application 63/252,818, filed October 6, 2021, the entirety of each of which applications is hereby incorporated by reference herein as if fully set forth herein.

TECHNICAL FIELD

The present invention relates to methods and kits for providing high throughput quantitative analysis of impact (e.g., by application of materials which affect - positively and/or negatively - microbial species) on human microbiome

BACKGROUND OF THE INVENTION

A bewildering array of microorganisms inhabit the human body including viruses, bacteria, fungi and protozoa (individually and collectively, they are referred to as "microorganism(s)" and "microbial"; a mixture of such microorganisms or species of such microorganisms are referred to as "polymicrobial"). Most co-exist in commensal relationship within the host (as microbiome) where they benefit from access to host-provided nutrients. Some microorganisms engage in mutually beneficial relationship with the host while others illicit pathogenic responses that can manifest in infectious outcome based on the host's level of immune strength. As used herein the term microbiome includes human, mammal, non-human/non-mammal microbiomes and microbial consortium(s).

The human microbiome plays an important role in shaping the trajectory of human health. An imbalanced microbiome on the skin can lead to eczema, psoriasis, acne, chronic wound infections, and malodor. In the oral cavity, it can lead to gum inflammation, bleeding, destruction of the gum and jawbone, malodor, dental cavities, sores, abscesses as well as systemic infections. In the gut, an imbalanced microbiome is associated with colon cancer, mood disorder, irregular food digestion and even decreased sleep.

Managing a healthy microbiome is important as some microorganisms help shape immunity, and supply nutrients that the human body cannot produce naturally while others provide such benefits as regulation of blood pressure and food digestion.

Understanding the impact of consumer health products on the human microbiome is important to determine how, and to what extent, these products provide health benefits. Before the advent of DNA sequencing technology, culture-based methods were employed using individual species of microorganisms at a time. However, not all microorganisms are capable of growth in a lab. In fact, it is estimated that less than 10% of all bacteria can even be cultured in the lab. Moreover, the use of select microorganisms in the lab in isolation from the rest of its community as a proxy to understand product efficacy can be misleading.

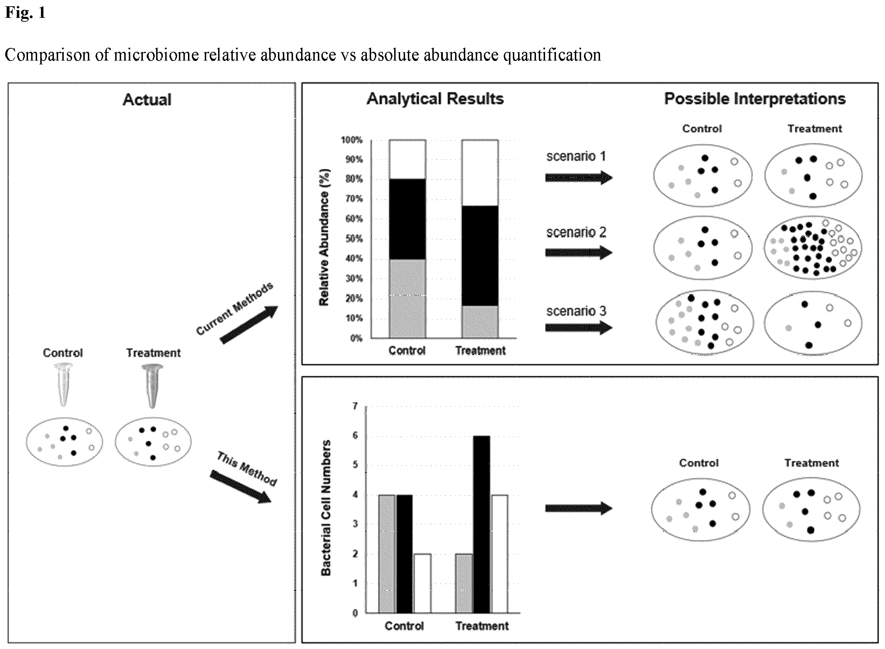

There are many microbiome profiling techniques employing different chemistries with different results, but DNA based profiling can be performed using 16S rRNA amplicon sequencing or the shotgun metagenome sequencing technique. These techniques amplify DNA extracted from samples as starting materials and, in the case of bacteria, produce a list of bacteria and their abundances (expressed in percentages) relative to each sample's total abundance. By themselves, these relative abundance (or percentage values), are not quantitative and cannot be used to evaluate changes in the microbiome. For example, in Fig. 1, a treatment may promote some species of bacteria to grow in abundance (as shown by the white colored graphic and bacterial depictions), inhibit the growth of others (as shown by the gray colored graphic and bacterial depictions) or have no (or minimal) population effects (as shown by the black colored graphic and bacterial depictions). The bacterial percentage data generated with current (i.e., non-quantitative) amplification methods can misrepresent data showing increases in bacterial population (as shown by the white graphic and bacterial depictions in scenario 3), population decreases (as shown by the gray graphic and bacterial depictions in scenario 3) or no (or minimal) changes in population (as shown by the black graphic and bacterial depictions in scenario 2) because the total abundance of bacteria for each sample is removed from consideration. In contrast, the methods of the present invention provide an efficient and accurate method of quantifying bacteria using DNA based microbiome profiling techniques.

Among the thousands of genes present in the genome of bacteria, the 16S rRNA gene represents an ideal biomarker to identify and differentiate bacterial species. This gene spans a length of 1600 nucleic acids. Amplicon sequencing employs universal pairs of primers designed to specifically target a portion of this bacterial 16S rRNA gene. The sequence of nucleic acids making up this gene is analyzed and compared against a curated database of 16S rRNA genes to identify individual bacterial species. While amplicon sequencing is affordable, it offers only a limited ability to identify bacteria down to the species level due to its inability to focus beyond a limited range of a targeted region in the 16S rRNA gene. For this reason, microbiome profiling using 16S rRNA amplicon sequencing is limited to only the genus taxonomic level and is unable to provide meaningful differentiation of bacteria such as their impact on human health or usefulness for biotechnological application.

Shotgun metagenome sequencing, on the other hand, analyzes all DNA materials in the samples using massively parallel processing capability and permits reconstruction of the entire genetic information using computer assisted analytical methods. While more costly, this method, unlike 16S rRNA amplicon sequencing, provides the means to identify microorganisms such as bacteria down to the species and strain levels as well as evaluating their genetic capabilities and their potential impact on human health or usefulness for biotechnological application.

The present inventor has found that by: quantifying and identifying microbial species in a human or mammalian subject's (or a non-human/non-mammalian) microbiome and understanding which microbial species are associated with (mapped to) a specific health (or healthcare) outcome (i.e., positive and/or negative health effects arising from the presence of particular microbial species), consumer products can be screened or assessed for their capability to provide health benefits by negatively or positively affecting particular microbial species population from a qualitative and absolute quantitative perspective.

Accordingly, an aspect of the present invention is to provide a method or kit for determining the quantities of specific microbial species present in the microbiome of human and/or mammalian subjects or non-human or non-mammal sources.

Another aspect of the present invention is to provide a method or kit for mapping (or associating) specific microbial species for oral care application (e.g., mapping species with respective health (or health care) outcomes).

A further aspect of the present invention is to provide a method or kit for screening and/or assessing proposed or currently marketed consumer products for capability of providing potential healthcare benefits based on the impact of such products on human, mammalian and/or non-human/non-mammal microbiome from a qualitative and absolute quantitative perspective.

DESCRIPTION OF FIGURES

Fig. 1 is an example illustrating differences between the microbiome relative abundance and absolute quantification methods.

Fig. 2 is a schematic showing parts 1 (Microbial Abundance Quantification) and 2 (Identification and Health Outcome Classification of Species) and, optionally, further comprise part 3 (Healthcare Product Screening vs. Health Outcome Associated Species) of the methods of the present invention, including their associated steps.

Fig. 3 is an example of a standard calibration curve (output abundance vs input DNA amount) used in calculating genomic DNA weights.

Fig. 4 is an example illustrating the data structure for performing bacterial species mapping to such oral health conditions.

Fig. 5 shows an excerpt of the database structure for oral microbiome bacterial species mapping metadata.

Fig. 6 shows an example bacterial species classification derived from mapping metadata for oral care application.

Fig. 7 shows alpha diversity measures assessing clinical microbiome impact of commercial mouthwash.

Fig. 8 shows the beta diversity ordination illustrating the efficacy of commercial mouthwash at restoring diseased microbiome to healthier levels.

Fig. 9 shows reductions in the total microbial load (i.e., total number of bacteria in the plaque) of oral plaque microbiome after using commercial mouthwash.

Fig. 10 shows the clinical microbiome impact of twice daily use of oral mouthwash on the abundances of bacteria associated with oral disease condition.

Fig. 11 illustrates heatmap representing cellular numbers for each species for oral care application.

Fig. 12 illustrates heatmap showing changes in bacterial cellular numbers compared to baseline for oral care application.

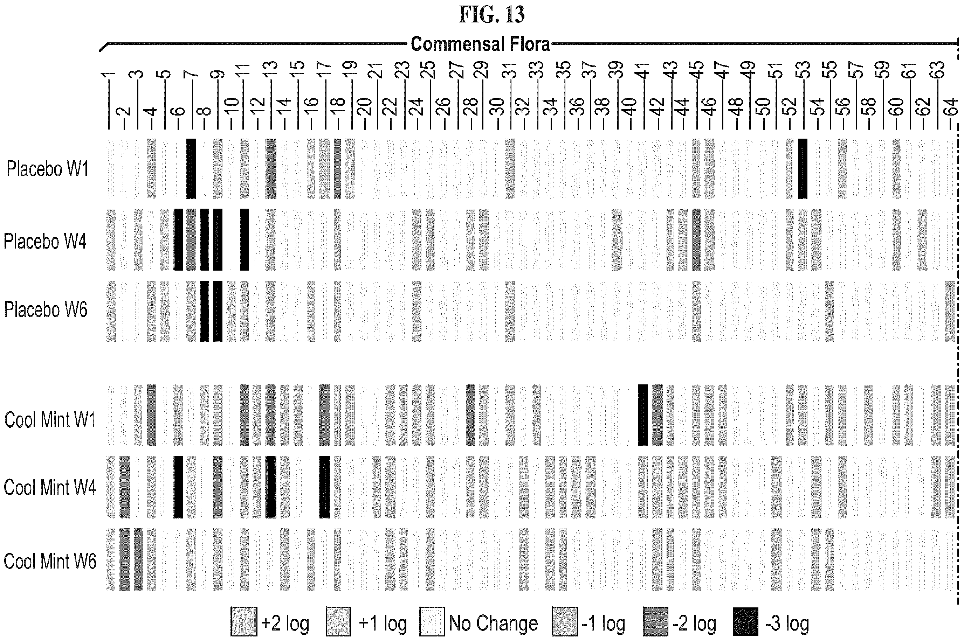

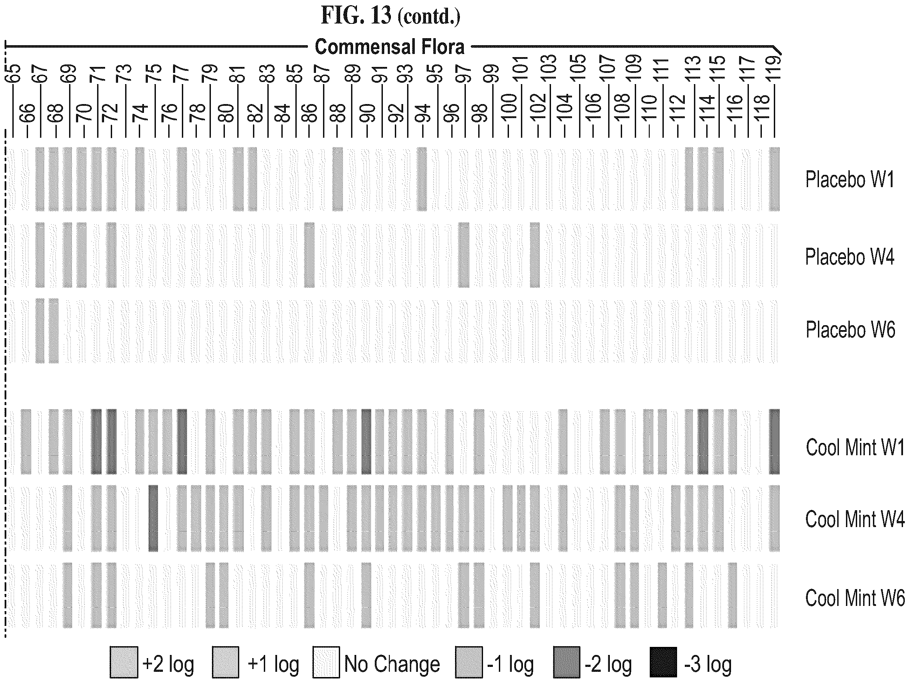

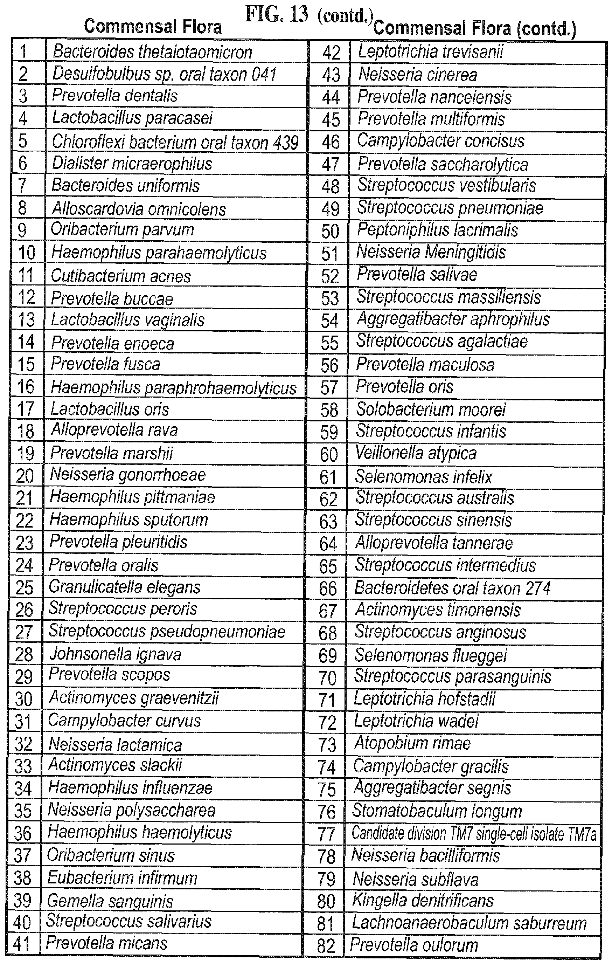

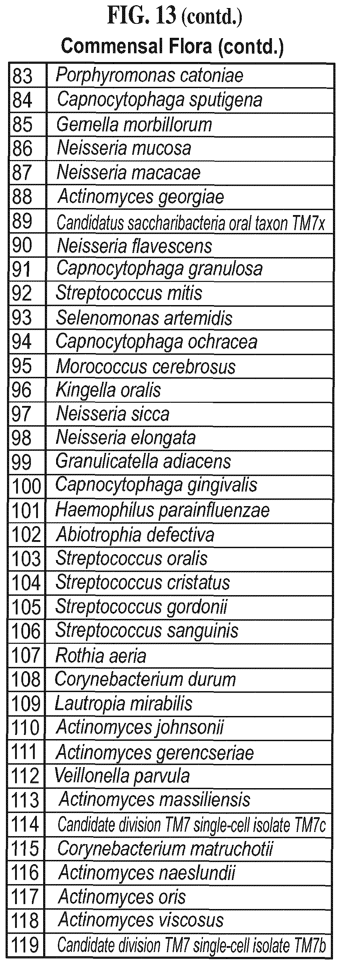

Fig. 13 shows excerpt of heatmap in Fig. 12, enlarging the "Commensal Flora" portion of the figure.

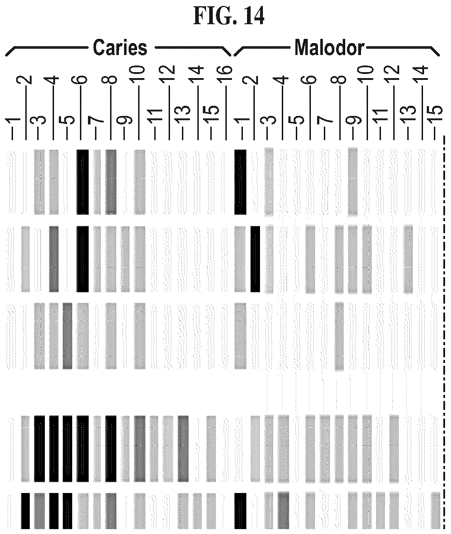

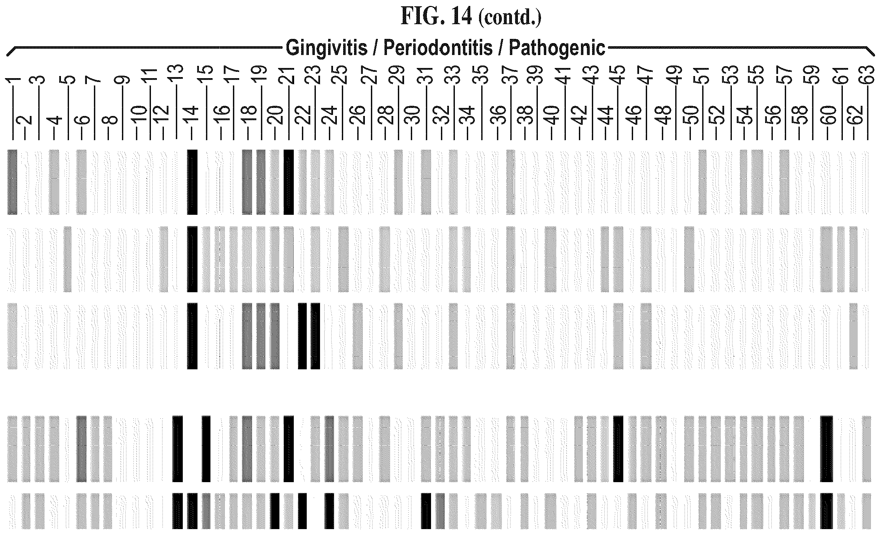

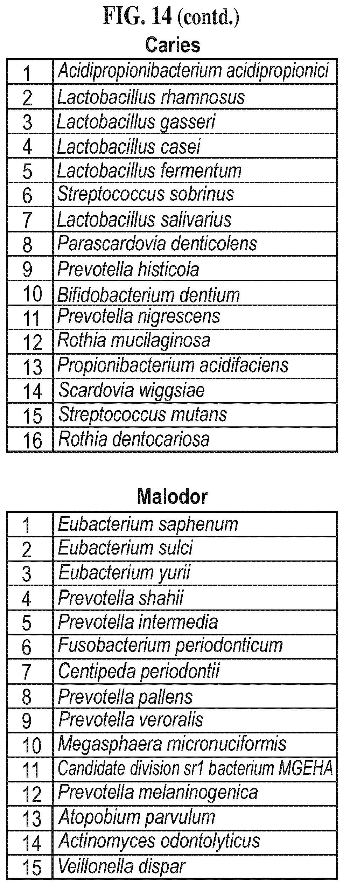

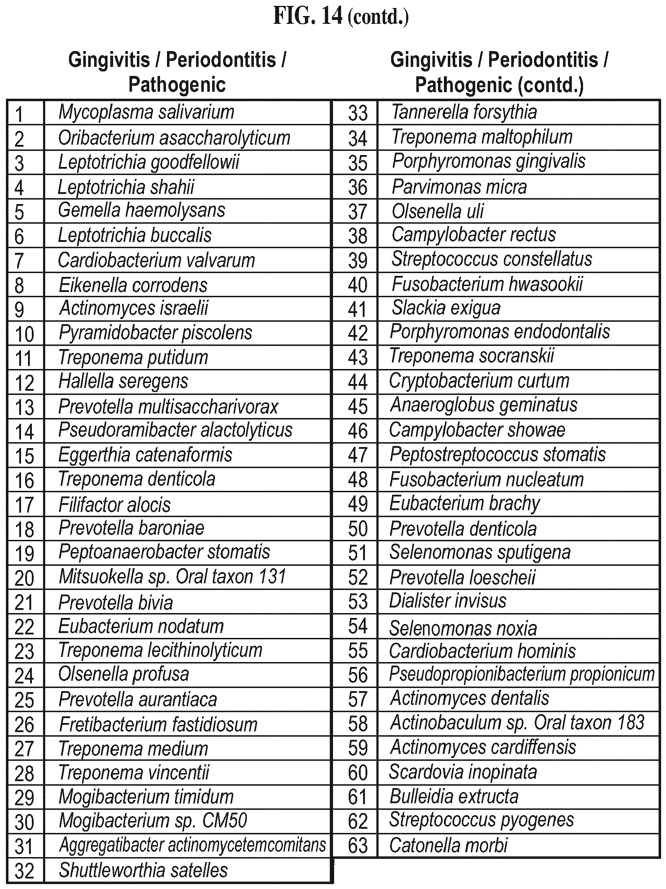

Fig. 14 shows excerpt of heatmap in Fig. 12, enlarging the "Caries, Malodor, and Gingivitis/Periodontitis/Pathogenic" portion of the figure.

SUMMARY OF THE INVENTION

In one embodiment, the present invention relates to a method for analyzing the microbiome of a human, mammal or non-human/non-mammal, comprising the steps of:

a. collecting at least one polymicrobial sample containing nucleic acid ("NA") material such as DNA or RNA from the human, mammal or non-human/non-mammal;
b. adding spike-in control standards to the sample of step a, which standard comprises at least two known microbial species not residing on/in the human, mammal or non-human/non-mammal, respectively, at known concentrations;
c. extracting NA from the microbial sample of step b;
d. analyzing the sample of step c using shotgun metagenome sequencing, wherein the technique provides comprehensive identification of the microbial organisms down to the species or strain taxonomic level and their abundances in the form of NA copy numbers or relative abundances;
e. calculate the cell numbers (or quantity or amount) for each of the microbial organisms identified in step d; and
f. mapping (or associating) on 1:n basis each microbial species identified in step d with a human and/or mammal health condition (or cause of the health condition in the case of the non-human/non-mammal source) based on known clinical testing information (including information obtained through calculation or extrapolation of relevant regional genomic or metabolomic measurements from a human and/or mammal) regarding the microbial species and the health condition (or associated cause).

In another embodiment, the present invention relates to a method for analyzing the microbiome of a human, mammal or non-human/non-mammal, comprising the steps of:

a. providing a sample collection kit for a user to collect at least one polymicrobial sample containing nucleic acid ("NA") material such as DNA or RNA from the human, mammal or non-human/non-mammal;
b. adding spike-in control standards to the sample of step a, which standard comprises at least two known microbial species not residing on/in the human, mammal or non-human/non-mammal, respectively at known concentrations;
c. extracting NA from the microbial sample of step b;
d. analyzing the sample of step c using shotgun metagenome sequencing, wherein the shotgun provides comprehensive identification of the microbial organisms down to the species or strain taxonomic level and their abundances in the form of NA copy numbers or relative abundances;
e. calculate the cell numbers (or quantity or amount) for each of the microbial organisms identified in step d; and
f. mapping (or associating) on 1:n basis each microbial species identified in step d with a human and/or mammal health condition (or cause of the health condition in the case of the non-human/non-mammal source) based on known clinical testing information (including information obtained through calculation or extrapolation of relevant regional genomic or metabolomic measurements from a human and/or mammal) regarding the microbial species and the health condition (or associated cause);
g. obtaining current and historical health data on the user or source related to the health condition (or cause of the health condition in the case of the non-human/non-mammal source) of step f;
h. determining a user or source reference population, the determination comprising the steps of:

i. identifying a plurality of human individuals, mammals or non-human/non-mammal sources having at least one common characteristic (such as reside in the same geographic region, included in same population age group, have same gender, practice same living habits);
ii. collecting at least one polymicrobial sample containing NA from the human individuals, mammals or non-human/non-mammal sources in the user or source reference population; and
iii. repeating steps b through g for each individual, mammal or non-human/non-mammal sources in the user or source reference population;
iv. determining the quantitative impact of a product or technology on the health condition of healthy human individuals in the user or source reference population related to the trait; and

i. comparing the data from step f. for the user with the data of step h. for the user or source reference population.

In another embodiment, the present invention relates to a method for screening a compound and/or formulation for impact on a human, mammal or non-human/non-mammal source microbiome comprising the steps of:

a. analyzing the microbiome of the human, mammal or non-human/non-mammal source, comprising the steps of:

i) collecting at least one polymicrobial sample containing nucleic acid ("NA") material such as DNA or RNA from a bodily region on the human, mammal or from the non-human/non-mammal source wherein the sample is a first sample;
ii) adding spike-in control standards to the sample of step a, which standard comprises at least two known microbial species not residing on/in the human, mammal or non-human/non-mammal source, respectively at known concentrations;
iii) extracting NA from the microbial sample of step b;
iv) analyzing and the sample of step c using shotgun metagenome sequencing, wherein the shotgun provides comprehensive identification of the microbial organisms down to the species or strain taxonomic level and their abundances in the form of NA copy numbers or relative abundances;
v) calculate the cell numbers (or quantity or amount) for each of the microbial organisms identified in step d; and
vi) mapping (or associating) on 1:n basis each microbial species identified in step d with human and/or mammal health conditions based on known clinical testing information (including information obtained through calculation or extrapolation of relevant regional genomic or metabolomic measurements from a human and/or mammal) regarding the microbial species and the health condition;

b. analyzing the impact of a first formulation or compound on the microbiome of the human, mammal or non-human/non-mammal source of step a) comprising the steps of:

A. administering the first formulation or compound to the bodily region of interest on the human or mammal or to the non-human/non-mammal source, and
B. repeating the steps a.(i) through a.(vi) for a second sample; and

c. comparing the data from step a.(vi) for the first sample with the data of step b. obtained upon completing the repeat of step a.(vi) for the second sample.

Detailed Description of the Invention

The methods and kits of the present invention can comprise, consist of, or consist essentially of the essential elements and limitations of the invention described herein, as well any of the additional or optional features, components, or limitations described herein.

The term "comprising" (and its grammatical variations) as used herein is used in the inclusive sense of (and, interchangeably with the terms) "having" or "including" and not in the exclusive sense of "consisting only of."

The terms "a" and "the" as used herein are understood to encompass the plural as well as the singular. Also, as used herein, "a," "an," "the," "at least one," and "one or more" are used interchangeably. Thus, for example, a composition that comprises "an" element can be interpreted to mean that the composition includes "one or more" such elements.

All percentages, parts and ratios are based upon the total weight of the composition of the present invention, unless otherwise specified.

All such weights as they pertain to the listed ingredients provided by way of example are based on the active level and, therefore, do not include carriers or by-products that may be included in commercially available materials, unless otherwise specified.

The term "concentration(s)" as used herein, in addition to its ordinary meaning can mean, include or be interchangeable with such other similar terms as amounts, weights, optical density (OD), bacterial colony forming units (CFUs), quantities, cell numbers and the like.

All documents incorporated herein by reference in their entirety are only so incorporated to the extent that the disclosure therein is not inconsistent with this specification.

In certain embodiments, the present invention as disclosed herein may be practiced in the absence of any compound or element (or group of compounds or elements) which is not specifically disclosed herein.

Unless defined otherwise, all technical and scientific terms used herein have the same meaning as commonly understood by one of ordinary skill in the art to which the invention belongs.

The term "Amplicon" as used herein means the product of a polynucleotide amplification reaction. That is, it is a population of polynucleotides that are replicated from one or more starting sequences. Amplicons may be produced by a variety of amplification reactions, including but not limited to polymerase chain reactions (PCRs), linear polymerase reactions, nucleic acid sequence-based amplification, rolling circle amplification and like reactions (see, e.g., U.S. Pat. Nos. 4,683,195; 4,965, 188; 4,683,202; 4,800,159; 5,210,015; 6,174,670; 5,399,491; 6,287,824 and 5,854,033; and US Pub. No. 2006/0024711, all of which are specifically incorporated by reference herein).

The term "relative abundance", as used herein, means the amount (or quantity) of a microbial taxon (such as a bacterial species) relative to the sum of the amounts of all taxa detected in a collected sample.

The term "absolute quantification" as used herein, means the absolute quantity of a single microbial target in a sample.

The term "species", as used herein, means one of the smallest (i.e., least inclusive) determinant of taxonomic groupings, ranking below a genus. In this sense, a species is a category of organisms which displays a high degree of mutual similarity within a genus.

The term "strain", as used herein, means a genetic or phenotypic variant (e.g., of a species), or a subtype or culture of a biological (e.g., microbial) species. Strains can arise, and exhibit character variation, due to, among other things, the effects of mutation, plasmid transfer, and/or recombination.

The phrase "clinically relevant" or "clinically significant" or "clinically important", as used herein with respect to impact on the microbiome, means that the impact has practical importance in producing a noticeable reduction in degree (e.g., severity) of symptoms of a health condition or prevention of onset (from exposure to pathogen) of symptoms of a health condition.

The term "health condition(s)" and/or "heath outcome", as used herein, means health conditions that negatively impact the overall health of an individual, such as various disease states. The term "disease state or condition" encompasses health disorders (i.e., a state in which the subject may not have any outward signs or symptoms of the disease or disorder but will develop the disease or disorder in the future), pre-disease state or precursor state of a disease; and such state in which the subject has a stage of the disease or disorder (e.g., an early, intermediate or late stage of the disease or disorder). In other words, the health condition or disease state is a spectrum that encompasses a continuum regarding the decrease in the overall health of a subject with respect to a disease or disorder. Included within the meaning of the terms "health condition(s)" and/or "heath outcome" are the cause(s) of such "health condition(s)" and/or "heath outcome" such as bacterial, fungal or viral causes as well as causes related to the presence (or absence) of certain materials (e.g., vitamins, minerals, enzymes or other materials) capable of generating or facilitating biochemical reactions in humans and/or mammals.

The term "healthy", as used herein, describes a human or mammal having no feeling of sickness (or showing symptoms of sickness) or diagnosed disease, disorder, infirmity, or ailment, more particularly a disease, disorder, infirmity or ailment which is known to elicit an immune response (such as fever), impair or otherwise diminish any of the five senses of such human or mammal, impair the general sense of well-being of such human or mammal or reduce the ability and/or desire of such human or mammal to engage, or otherwise discourage (through pain or reduced of control) such human or mammal from engaging, in normal every-day activities such as, eating, standing, thinking, seeing or moving.

The term "nucleic acid" refers to deoxyribonucleotides or ribonucleotides and polymers thereof in either single- or double-stranded form, made of monomers (nucleotides) containing a sugar, phosphate and a base that is either a purine or pyrimidine. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues. The terms "nucleic acid," "nucleic acid molecule," or "polynucleotide" are used interchangeably and may also be used interchangeably with gene, cDNA, DNA and/or RNA encoded by a gene.

The term "baseline microbiome measurement", as used herein, means a measurement of the microbiome taken at a time before any type of treatment intervention or application of an active agent, including application of health care benefit agent such as an antimicrobial agent. The "baseline microbiome measurement" can also mean a "reference microbiome measurement"

The term "benchmark microbiome measurement", as used herein, means a measurement of the microbiome taken after application of a known industry standard (e.g., industry standard active agent for killing microorganisms), which measurement is used in ranking an "experimental microbiome measurement(s)" (defined below) versus the benchmark microbiome measurement to determine if such experimental microbiome measurement falls higher, lower or on par with such benchmark microbiome measurement. The measurements observed versus "benchmark microbiome measurement" include negative results and results observed after termination of product or technology used in obtaining "experimental microbiome measurement" (defined below).

The term "experimental microbiome measurement", as used herein, means a measurement of the microbiome taken after application of active ingredient or health care technology, which measurement is used to determine the level of benefit/detriment to such microbiome resulting from the active agent relative to a benchmark microbiome measurement and/or a baseline microbiome measurement, including for determining whether the experimental microbiome measurement falls higher, lower or on par with respect to such benchmark microbiome measurement and/or baseline microbiome measurement, based on a given measurement criteria.

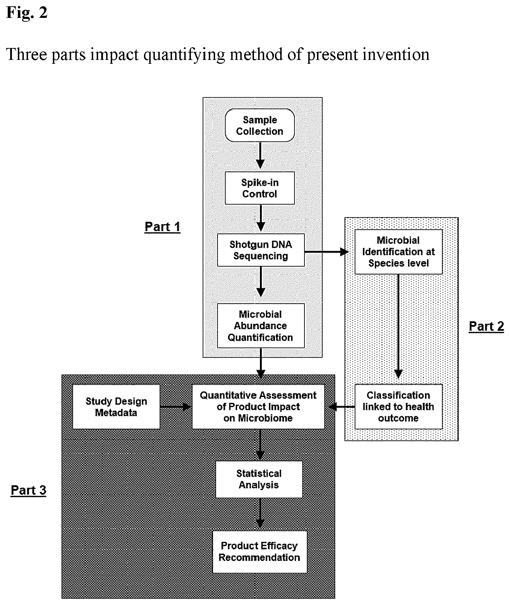

As shown in Fig. 2, the methods of the present invention comprise parts 1 (Microbial Abundance Quantification) and 2 (Identification and Health Outcome Classification of Species) and, optionally, further comprise part 3 (Healthcare Product Screening vs. Health Outcome Associated Species).

## Part 1 - Microbial Abundance Quantification

The methods of the present invention further comprise the steps associated with the Microbial Abundance Quantification (Part 1).

As an initial step of the Microbial Abundance Quantification (Part 1) process, microbiological samples are obtained from human or mammalian subjects (or non-human/non-mammalian sources) containing respective nucleic acid ("NA") material such as DNA or RNA.

During sample processing, all NA materials undergo several rounds of amplification, which results in the scalar or quantile information about microbial abundances to become lost. For this reason, all microbiome NA sequencing results are provided in terms of relative abundances of specific types of microorganisms over the total microbial NA analyzed per sample.

Spike-in Control Step

The Microbial Abundance Quantification (Part 1) of the present invention also comprises the step of adding controlled amounts of NA from microorganisms (such as bacteria) or controlled amounts of microorganisms themselves not normally present on/in the human, mammal or non-human/non-mammal, respectively, from whom/which the samples are collected; this step is known as the "spike-in" control. The spike-in method used in the present invention is distinct from the "microbiome mock community standard" as described in Fouhy, F., Clooney, A.G., Stanton, C. et al. 16S rRNA gene sequencing of mock microbial populations- impact of DNA extraction method, primer choice and sequencing platform. BMC Microbiol 16, 123 (2016). While the microbiome mock community standard is intended to diagnose and optimize the experimental reproducibility of NA based microbial profiling techniques from one experiment to another, the methods of the present invention use the spike-in control to enable the absolute quantification of microbial (such as bacterial) cell numbers. The spike-in control comprises microorganisms or NA that can be added to analytical specimens (e.g., as collected from various bodily regions/sites of interest, such as oral cavity, skin, wound sites and gut, of test subjects as test samples; or as taken collected from a non-human/non-mammal source) as a quantifiable standard. In preferred embodiments, the spike-in control comprises at least two species, preferably at least three species, of microorganisms or NA, and the spike-in control is then analyzed, together with the samples. Adding in controlled amounts of spike-in control material as a quantifiable standard, aids in quantifying the microbial cell numbers. Specifically, the spike-in control material helps translate amplified NA copies into microbial cell numbers. In certain embodiments, the spike-in control comprise viral, bacterial or fungal species. Preferably, the spike-in control comprises bacterial species.

Further, the spike-in control method described in the present invention provides a more accurate means to quantify microorganisms from NA based microbial profiling as compared to approximation techniques. Examples of such approximation techniques include quantifying the total microbial count of each sample using 16S rRNA quantitative PCR, culture, microscopy, fluorescence or bioluminescence based microbial enumeration and multiplying the relative abundances to approximate the absolute quantities of identified microorganisms such as described by Jian C. et al. in "Quantitative PCR provides a simple and accessible method for quantitative microbiota profiling" in PLoS ONE 15(1): (2020). Often these approximation techniques result in the compounding of errors associated within the first steps of quantifying the total microbial count such as introducing non-specific amplifications, mispriming or priming inefficiencies caused by degenerate NA sequences in 16S rRNA universal primers, or the selective nature of laboratory culture media for growth of microorganims or lack of linearity between fluorescence readouts (or values as per conventional spectrophometric or fluorometric measurements) to the actual total microbial count.

Providing the spike-in control NA material circumvents loss of information by allowing the construction of standard calibration curves (input control NA vs output relative abundance) which allows the calculation of abundances for individual species of microorganisms within each sample.

The amount of spike-in control which is to be directly added to each collected sample should not exceed 20% of the total microbial load contained in any collected sample. In preferred embodiments, the NA material is inactivated microbial cells or DNA.

NA Extraction Step

The Microbial Abundance Quantification (or Part 1) process also comprises the step of extracting NA material from the collected samples, including the spike-in control. NA extraction methods for DNA and/or RNA are well-known to skilled artisans and any of these methods, many in the form of commercially available NA isolation kits or non-standardized manual in-house methods, can be used to extract or isolate the NA material in the methods of the present invention. Examples of suitable DNA isolation kits include, but are not limited to, DNeasy Powersoil Pro kit (Qiagen Inc, Germantown MD, catalog# 47014), ZymoBIOMICS DNA kit (Zymo Research, Irvine CA, catalog# D4300), or QIAamp DNA mini Kit (Qiagen KK.). Useful isolation techniques include, but are not limited to, lysis, heating, alcohol precipitation, salt precipitation, organic isolation, solid phase isolation, silica gen membrane isolation, CsCl gradient purification, or any combination thereof. In preferred embodiments, the microbial samples, including spike-in controls, are lysed using chemical (treatment with acid or base or enzyme) and/or physical means (French press, vortexing or bead beating) to generate a crude extract containing various molecules (carbohydrate, lipid, protein, and nucleic acid). By this stage, all live microorganisms are destroyed and reduced down to their constituent macromolecules. DNA is purified through ion exchange chromatography on silica resins and eluted in appropriate sterile buffer solution for further processing. In the case of RNA extraction, DNA is degraded (e.g., by a DNase enzyme) after NA extraction (as described above) and the RNA is reversed transcribed to cDNA.

Shotgun Sequencing Step

The Microbial Abundance Quantification (Part 1) also comprises the step of shotgun sequencing to identify and differentiate individual species or strains of microorganisms in the collected sample.
All nucleic acid material (NA) extracted from the samples undergo "Shotgun" metagenomic sequencing to enable analysis of nucleic acid sequences. Specifically, shotgun sequencing comprises fragmenting NA into manageable sizes and fusing them with bar-coding adapter sequences and then, amplifying them to generate a library of NA fragments. The samples are normalized and loaded into a flow cell device of the sequencer. As each of the NA fragments gets replicated one nucleic acid position at a time, different fluorescence light signals are emitted then recorded. The millions of NA sequences thus generated are then quality filtered, assembled, then compared against curated databases to identify the types of species of a microorganism and the number of amplified NA copies detected. Shotgun sequencing is also discussed in US Patent Publication 20130059737A1 to Drmanac et al. US Patent Publication 20180135120A1 to Ruan and US Patent Publication 20180330044A1 to Lawley et al., each of which patent documents are herein incorporated by reference in its entirety.

Microbial Abundance Quantification Step

The Microbial Abundance Quantification (Part 1) further comprises the step of constructing a standard calibration curve for each sample by plotting the input amount of spike-in NA for each control species on the x-axis vs the corresponding relative abundances on the y-axis for obtaining a linear regression formula. The linear regression formula thus obtained is applied to the relative abundance of all species of a microorganism (e.g., bacteria) in the sample other than the spike-in control microorganism to obtain their total weights of NA. To calculate the number of cellular abundances, the total NA weights are divided by their genome molecular weights obtained from curated genome databases (GenBank, RDP, Silva etc.) and the resulting molar numbers are multiplied by Avogadro's constant to obtain the cellular abundances of the microorganism expressed as Calculated Microbial Units (or "CMUs").

**Part 2 - Identification and Health Outcome Classification of Species**

The methods of the present invention further comprise the steps associated with the Identification and Health Outcome Classification of Species (Part 2).
Notably, the list of microorganism (e.g., bacterial) species or taxa generated from shotgun sequencing are often provided to the end-user "as is" and provides little to no context of or commentary regarding their potential association with or meaningful impact to human health.
The Identification and Health Outcome Classification of Species (Part 2) of the present invention comprises the step of generating and employing a heath outcome classification scheme (or "mapping") for the identified microbial species to resolve this "no context of or commentary" gap in knowledge. The "mapping" is accomplished by reviewing and comparing the list of microorganisms identified from shotgun sequencing at the species or strain taxonomic levels, in the context of the scientific literature including primary scientific articles and clinical reports, to assign a classification group according to their health impacts.
Specifically, by mapping microbial species to oral/skin diseases, the specific species of microorganism (e.g.,

bacteria), the number microorganisms or microbial species and/or the percentage of the microbiome as a whole contributing to specific health conditions (or cause of such health conditions as might be found in a non-human/non-mammal source of microorganisms and/or associated microbiome) can be determined and, as a consequence, the clinical relevance of the data enhanced (i.e., by the mapping process's exclusion, from such data, of transient contaminants and/or unnamed microbial taxa).

For example, in oral care such impacts include but are not limited to:

- Commensal
- Gingivitis
- Periodontitis
- Caries/Acidogenic
- Malodor
- Oral pathogens
- Systemic pathogens

Microbial species that are not part of the normal resident human and mammalian flora and have low abundances below $10^3$ CMUs are assigned to the group of "Transient Contaminants" and those unnamed microbial taxa without scientific names and no prior scientific knowledge of their impact on human health are assigned to the group of "Unknowns". Such microorganisms are considered spurious and, for refinement purposes, are not considered in the quantification processes of the present invention.

Without being by theory, the present inventor has discovered that by administering the data refinement steps described above, any product and/or technology impact assessment on human microbiome becomes solely dependent on those microbial species that are most relevant to the health outcome being evaluated. This improves the sensitivity and clinical relevance of the generated results - which also aligns well with standardized microbiological assessment methods such as conventional colony counting assays. An added benefit of administering this data refinement step(s) is the reduction in the false positive discovery rate of microbial species impacted by a product/technology as opposed to conventional methods of analysis which are prone to detecting transient colonizers with no meaningful clinical relevance.

Alternatively, the exclusionary refinement principles outlined above for Transient Contaminants and Unknowns can be applied to (i.e., so as to exclude from the methods of the present invention) taxa that are well known to reside (and/or studied as residing) in a microbiome of interest for the purpose of focusing assessment (e.g., the research questions or objectives) on Transient Contaminants and Unknowns to look for impact (relative or none) on the microbiome. The objectives of such assessments can include identification of previously unknown markers for disease or health benefit providing microorganisms like probiotics.

**Optional Steps/Components**

Part 3 - Healthcare Product Screening vs. Health Outcome Associated Species

The methods of the present invention, optionally, comprise the steps associated with the screening of healthcare products vs. health outcomes associated species (Part 3) to determine, in view of the positive and/or negative effects of such products on respective microbial species, the potential health care outcomes likely resulting from such products.

The Healthcare Product Screening vs. Health Outcome Associated Species (Part 3) involves the use of study metadata such as the different product types, length of product use, subject demography or clinical parameters and any other relevant information to evaluate how they impact the human microbiome.

Microbiome Diversity Analysis

The populations of microorganisms constituting the human, mammal or non-human/non-mammal microbiome is described in the scientific art in terms of alpha and beta diversities.

For purposes of the present invention, alpha diversity comprises a measure of observed richness (number of distinct microbial species) and their evenness of (i.e., the extent to which the abundance of species is evenly distributed in view of the) overall diversity (also known as the Shannon-Weaver diversity index in a biological sample).

The alpha diversity analysis of (or as modified by refinement step) the present invention comprises the following steps:

a) removing Transient Contaminants and Unknown taxa from the microbiome profiling data to ensure the results have real-world relevance, and

b) determining the alpha diversity values of the microbiome present in a sample (e.g., oral care sample) from a tested individual or source or subject using a statistical programming language software such as R (open source) with the *'vegan'* package ver. 2.5-7 or under Python (open source) with QIIME ver. 2, and

c) comparing the alpha diversity values determined at step b) to a reference alpha diversity value and calculating the statistical significance using the analysis of variance followed by post-hoc analysis using the Tukey Honest Significant Difference test for simple study designs or linear mixed effects model with repeated measures for human clinical trials with $\alpha$=0.05.

The method according to the present invention comprises a step (c) of comparison of the alpha diversity value of a microbiome of a tested individual or source or subject, with a reference alpha diversity value. Preferably, the reference value will be calculated on a reference oral cavity microbiome profile according to the same method of calculation and/or based on the same diversity index.

The "tested individual(s) or source(s)" refers to an individual human or mammalian subject or non-human/non-mammal source from whom/which originates a biological sample that is tested for determining the alpha diversity value of the subject microbiome.

The beta diversity of a microbiome describes differences in the microbial compositions that exist between groups of samples such as one brought about by treatment with a product over a certain period of time. Of relevance to the present invention, this analysis permits the visualization of the microbiome composition shifts arising from the impact of treatment with healthcare products and qualitatively describes the extent to which a microbiome state has changed over the course of treatment such as the return of a diseased state microbiome or one at risk of developing a condition of interest to healthier levels such as defined by the absence of the clinical manifestations of the disease conditions.

The beta diversity analysis of (or as modified by) the present invention comprises the following steps:

a) removing transient contaminants and unknown taxa from the microbiome profiling data to ensure the results have real-world relevance, and

b) calculating the pairwise differences between groups of microbiome samples using the microbial abundance values obtained in Part 1 of this invention (expressed in CMUs) with the weighted UniFrac distance matrix computation algorithm within the context of microbial evolutionary relationships or Bray-Curtis dissimilarity or any such distance matrix computation algorithms using a statistical programming language software such as R (open source) with the *'phyloseq'* package ver. 3.13 or under Python (open source) with QIIME ver. 2, and

c) graphically representing microbiome status of the different groups of samples comparing the "tested individual(s) or source(s)" against a reference population in a two-dimensional or three-dimensional metric or non-metric graphical coordinate plane such as Principal Coordinate Analysis (PCoA) or Non-Metric Multidimensional Scale (NMDS), and

d) calculating the statistical significance of the microbiome shifts using the Permutational Analysis of Variance (PERMANOVA) with $\alpha$=0.05.

Microbiome Quantification

The total microbial load quantification facilitated by Part 1 of this invention is a high-level summarization to examine the impact of study design metadata. In much the same manner as manual enumeration of bacterial colony counting, the total number of microorganisms expressed in CMUs (from Part 1 - Microbial Abundance Quantification of the present invention) for each sample is calculated and transformed to Log10 values. The average of Log10 values for all samples is reported for each grouping in the study design metadata (product type, length of use, subject demographics, and clinical endpoints etc..) and statistical significance is tested using the analysis of variance followed by post-hoc analysis using the Tukey Honest Significant Difference test for simple study designs or using the linear mixed effects model with repeated measures for human clinical trials with $\alpha$=0.05.

For quantitative assessment of effect on human, mammalian or non-human/non-mammal microbiome, the impact of the study design metadata on the number of microorganisms associated with each of the different health outcome is evaluated. On a per sample basis, the cellular abundance, or CMUs (from Part 1 - Microbial Abundance Quantification of the present invention) of all species of microorganisms assigned to specific health outcome are aggregated (from Part 2 - Identification and Health Outcome Classification of Species of the present invention) and then compared against specific study design metadata criteria (from Part 3 - Healthcare Product Screening vs. Health Outcome Associated Species of the present invention). These results can be expressed: (1) in the form of bar graphs showing the total microbial load in CMUs and portions of these bars representing specific categories of microorganisms related to a specific health outcome (e.g., gingivitis/periodontitis in the case of certain bacteria) at a time or (2) in the form of bar graphs showing the microbial load in CMUs of only those species specific to a health outcome (e.g., bacterial species causing malodor) or (3) the latter in the form of boxplots comparing the average log10 transformed CMUs of all samples in the different study design metadata groups.

To illustrate the above microorganism-health outcome relationship and the relative magnitudes of certain parameters, a heatmap can be generated to assess the impact of study design metadata on individual species of microorganisms (e.g., bacteria). The term "heatmap" as used herein means a data visualization technique that shows magnitude or intensity of a phenomenon as corresponding color variation where the variation in color may be by hue or intensity, giving obvious visual cues to the reader about how the phenomenon is clustered or varies over space. The heatmap is generated using a statistical programming language software for graphical visualization/graphics such as R (open source), Python (open source) or MATLAB (MathWorks, Natick, MA), where the identities of microbial species are listed on the horizontal x-axis in alphabetical order or grouped according to clinical relevance. The different groups of samples such as those collected from the reference or test subjects or at different time points in the study are listed on the vertical y-axis. The data represented in such heatmap can include, for example, the (1) cellular number of each microbial species or a representation of their magnitude using log10 transformation or (2) difference in cellular numbers of bacteria representing specific microbial reduction or proliferation following a test condition (treatment, length of use etc.).

Applications of Methods of the Present Invention

Healthcare Technology Screening for Antimicrobial Properties

The methods or kits of present invention can be used to screen for personal care/healthcare technologies that have antimicrobial properties against select microbial species or the entire microbiome for the purpose of reducing the total microbial load for example oral care hygiene products to protect against caries, gingivitis, malodor and systemic illnesses related to oral health as described below or skin care applications (by application to, or otherwise targeting, the skin or distinct regions of the skin) to prevent against or reduce skin diseases (e.g., breakouts of eczema or acne) as well as to protect against opportunistic microbial infections in minor wounds.

Healthcare Technology Screening for Prebiotic and Probiotic Properties

The methods or kits of the present invention can be used to screen for personal care/healthcare technologies that have prebiotic or probiotic properties against select microbial species or the entire microbiome for the purpose of nurturing a balanced microbiome, sustaining the growth of select beneficial microorganisms (or microorganisms such as bacteria which work positively to aid function of the body region in which they reside - as opposed to harming such region or the entire body) or to restore select microorganisms impacted by a disease condition to normal levels commensurate as found in healthy human individuals (or mammals or non-human/non-mammal sources) spanning a wide range of product categories like skincare, oral care, wound care and digestible applications.

Diagnostics Tool for Personalized/Precision Care

The methods or kits of the present invention can be used as a direct-to-consumer diagnostic tool to evaluate the microbiome condition of the mouth, skin, or gut to provide (1) a simple to understand report showing the types and numbers of microorganism(s) as they relate to specific health conditions (2) an average of the healthy vs diseased population for comparison (3) impact of personal care products after specified length of use or (4) projected impacts based on recommended products. This application is distinct from currently existing personalized care models since it provides direct numerical abundance data of microorganisms in association with human health as opposed to providing semi-qualitative description of the microbiome composition as a fingerprint of what types of microorganisms and the relative proportions in which they are present.

**Examples**

Any methods or kits of the present invention as described in following examples illustrate specific embodiments of compositions of the present invention but are not intended to be limiting thereof. Other modifications can be undertaken by the skilled artisan without departing from the spirit and scope of this invention.

Example 1

Sample Collection for Microbiome Analysis

- Supra- or sub- gingival plaque and/or saliva were collected from clinical subjects by trained professional dentists to assess the impact of oral care products (including mouthwash, floss and dentifrices) on plaque forming or gum disease associated microbiota.

- Supragingival plaque samples were collected using sterile curettes by moving from mesiobuccal gingival margin to midbuccal, from distobuccal to midbuccal then repeated on the lingual side. Supragingival samples that were thus collected were pooled per subject basis and placed in 250 $\mu$L of sterile ultra-pure grade phosphate buffered saline, pH 7.2 in 1.5 mL microfuge tubes then immediately stored frozen at -80°C.

- Subgingival plaque samples were collected using several sterile paperpoints as needed by the aseptic use of sterile tweezers dipped below the gum margin and stored similarly to supragingival plaque samples.

- To assess for malodor associated oral microbiota, subjects' tongues were sampled in two locations with a single brush posterior to the circumvallate papillae and anterior in the middle of the tongue.

Addition of Spike-in Control and Extraction of DNA

To enable quantification of bacterial abundances, instructions were provided that the spike-in control bacteria be first added to each of the collected specimens. The spike-in control was to consist of a suspension of inactivated *Truepera radiovictrix*, *Imtechella halotolerans* and *Allobacillus halotolerans* bacterial cells - i.e., species not normally residing on/in humans, mammal, on non-human/non-mammal sources under consideration and have defined cell densities and genome sequencing data information. The instructed amount for addition was to be less than 20% of total microbial load. One species contains approximately $1 \times 10^6$ cells, followed by approximately $1 \times 10^5$ cells for the second species and $1 \times 10^4$ cells for the third species. ZymoBIOMICS Spike-in Control II (Low Microbial Load) is one such commercially available kit (Zymo Research, Irvine CA, catalog# D6321).

- Following the addition of the spike-in control, samples were centrifuged at max speed ~13,000 RCF for 10 minutes to pellet the microbial samples.

- The supernatant was carefully discarded after centrifugation without disturbing the microbial cell pellet.

- To improve the efficiency of hard to lyse bacteria, the pellet was resuspended in 180 $\mu$L of sterile ultra-pure grade phosphate buffered saline, pH 7.2 and 6 $\mu$L of metapolyzyme (Sigma-Aldrich, St. Louis MO, catalog# MAC4L-5MG) is added then incubated at 35°C for 12 hours.

- The microbial samples were then lysed using bead-beating and DNA was extracted then purified using silica-based mini column chromatography which are commercially available such as DNeasy Powersoil Pro kit (Qiagen Inc, Germantown MD, catalog# 47014) or ZymoBIOMICS DNA kit (Zymo Research, Irvine CA, catalog# D4300) following the manufacturer's instructions.

- The above process steps of this example can be automated using programmable automated liquid handling robotics system from HAMILTON ROBOTICS (Reno, NV) or BECKMAN COULTER (Brea, CA).

- All DNA samples were eluted in 30 $\mu$L DNase and RNase free molecular biology grade Tris buffer or phosphate saline buffer. All DNA samples were then quantified by fluorometry using 1 $\mu$L of each DNA samples in Qubit dsDNA HS assay kit (Thermo Fisher Scientific, Waltham MA, catalog# Q32851) and Qubit4 fluorometer instrument (Thermo Fischer Scientific, Waltham MA, catalog# Q33238) per manufacturer's instructions.

- Representative DNA samples were randomly selected and subjected for quality control check by analyzing 1 $\mu$L of DNA by capillary gel nano electrophoresis using the High Sensitivity DNA chips and reagents in BioAnalyzer or TapeStation instrument (Agilent Technologies, Santa Clara, CA).

- Eluted DNA are stored at 4°C for short term storage not exceeding 1 week or at -80°C for longer term storage.

Shotgun Sequencing Procedure

- DNA libraries (i.e., the pool of all possible DNA fragments extracted from the samples to be analyzed) were prepared as follows. Nextera XT DNA library preparation kit (Illumina, San Diego CA, Catalog# FC-131-1096) was used to fragment and amplify DNA samples and Nextera Index Kit (Illumina, San Diego CA, Catalog# FC-131-1001/2001/2002/2003/2004), to attach bar coding and sequencing labels (i.e., differentiate sample origins). Other DNA library kits are available and can be equally used in generating the DNA libraries of the present invention.

- The total genomic DNA isolated from each sample were diluted to a concentration of 0.5 ng/$\mu$L and 2 $\mu$L containing 1 ng of DNA was used as input material for DNA library preparation according to the manufacturer's instructions.

- The resulting DNA libraries (from the above process steps of this example) were purified to remove the spent reagents using AMPure XP magnetic beads (Beckman Coulter, Brea CA, Catalog# A63881) according to manufacturer's instructions then eluted in sterile DNase, RNase-free molecular biology grade Tris-Cl buffer.

- All DNA libraries were quantified by fluorometry using Qubit dsDNA HS assay kit and Qubit 4 fluorometer instrument similarly to DNA extraction quality control.

- Following DNA libraries preparation and quantification, samples were normalized for equimolar pooling then loaded onto a HiSeq DNA Sequencer (Illumina, San Diego CA) targeting 3 million of paired end 150 bp read depth using the HiSeq v3 sequencing chemistry reagents.

Microbial Abundance Quantification

- The above DNA sequencing data were preprocessed (Chen C, Khaleel SS, Huang H, Wu CH. Software for pre-processing Illumina next-generation sequencing short read sequences. Source Code Biol Med. 2014), and quality filtered using CosmosID Inc software for taxonomic identification of bacteria to the species and strain levels. The resulting bacterial taxonomy and abundance score tables are used as input data for further analysis using the R statistical programming language software ver 3.6.2.
- The list of bacterial species identified from all samples were evaluated against the taxonomy and genome databases provided at the National Center for Biotechnology Information (NCBI, *www.ncbi.nlm.nih.gov*). Taxonomy IDs and the mean genome sizes were recorded. Unidentified bacterial species and placeholder taxa with no known genome sizes were excluded from further analysis.
- The abundance scores were then converted to percentage values relative to each sample to enable construction of individual standard calibration curves.
- The slope and intercept of the linear regression for each sample are calculated using the input genomic DNA weight in ng of the spike-in control bacterial species on the y-axis and output relative abundance on the x-axis. The input genomic DNA weights are calculated as follows:

$$= \frac{\text{cell numbers} \times \text{genome size (bp)} \times \text{molecular weight of dsDNA (650g per bp)}}{\text{Avogadro's constant} (6.02 \times 10^{23} \; cell \; numbers \; per \; g )}$$

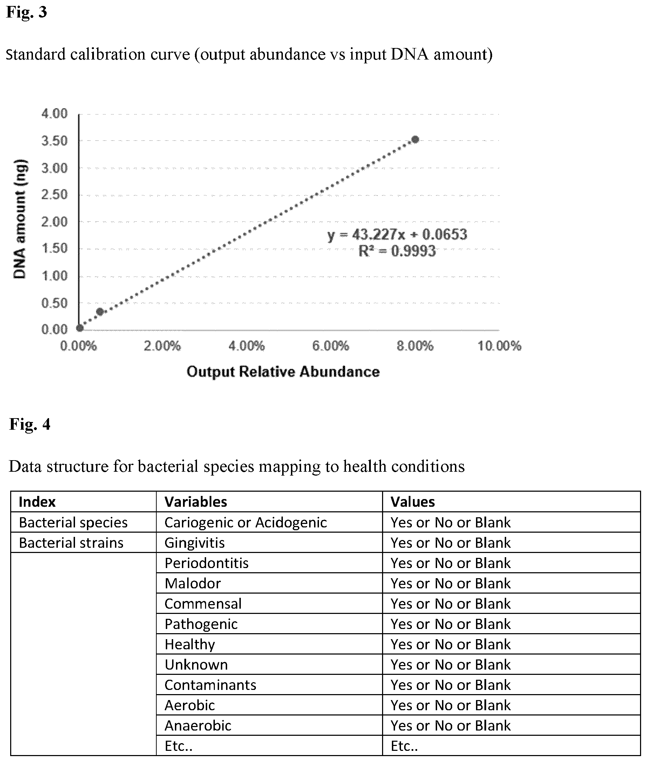

The graph of the standard calibration curve (output abundance vs input DNA amount) is provided in Fig. 3.
For example, if the number of spike-in control bacterial cells is $1 \times 10^6$ cells for species 1 with a genome size of 3,260,398 bp, this would result in a genome weight of 3.5 ng. Similarly, for species 2 with $1 \times 10^5$ cells and a genome size of 3,086,951 bp, this would result in 0.33 ng. For species 3 with $1 \times 10^4$ cells and a genome size of 2,726,708 bp, this would result in 0.029 ng. Plotting these values on the y-axis and output relative abundances on the x-axis (for example, 8%, 0.5% and 0.03% respectively for species 1-3) produces a linear regression with slope 43.23 and intercept 0.0653.
- The genomic DNA weights of all bacterial species identified in each sample were then calculated using the slopes and intercepts of linear regression using:

$$= \text{relative abundance for each species} \times \text{slope} + \text{intercept}$$

If a target bacterial species from the same sample as in the previous example has a relative abundance of 12%, this will translate to an input genomic weight of 5.25 ng.

- The resulting genomic DNA weights were converted to bacterial cell numbers as sampled from each individual subject using:

$$= \frac{\text{genomic DNA weights} \times \text{Avogadro's constant } (6.02 \times 10^{23} \; cell \; numbers \; per \; g)}{\text{genome size (bp)} \times \text{molecular weight of dsDNA (650g per bp)}}$$

If the said target bacterial species has a genome size of 4,135,403 bp, the number of bacterial cells in the sample will be 1,176,426 or $\log_{10}$ 6.07.
- The cellular abundance of all bacterial species or strains thus calculated were expressed in measures of Calculated Microbial Units or CMUs as the *in-silico* equivalent of bacterial Colony Forming Units.

Bacterial Species Mapping

- Many species of bacteria residing on the human body are implicated in multiple health and disease conditions. Therefore, a 1:1 distinct classification of each bacterial species and strains in association with human health conditions is impractical.

- Instead, a 1:n (or 1 : many) classification scheme is best adapted for mapping the role of bacteria in human microbiome in a comprehensive manner and used for purposes of the present example.

- Oral bacterial species can be mapped to a number of important oral physiological conditions as shown below. An example of the data structure for performing bacterial species mapping to such oral health conditions can be found in Fig. 4.

- Each of the bacterial species (or strains) identified from DNA sequencing were classified according to the metadata structure described above using published and peer-reviewed scientific literature and clinical study reports which can be accessed from PubMed database at NCBI (*pubmed.ncbi.nlm.nih.gov*). Other reputable scientific journals/journal sites may also be used.

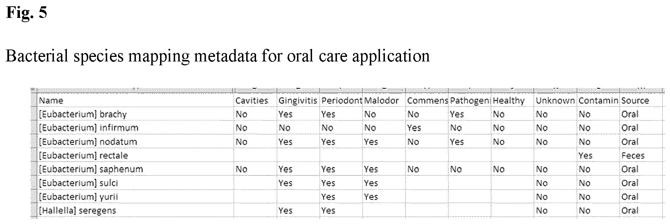

- An excerpt of a generated mapping document showing the mapping of certain oral bacterial species is provided in Fig. 5.

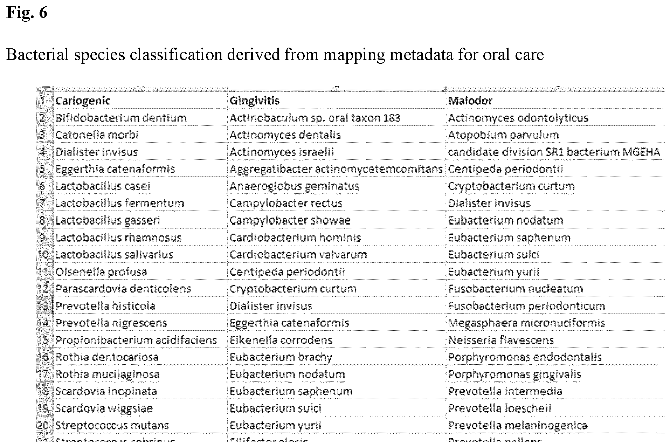

- The bacterial mapping metadata shown in Fig. 5 was further processed using statistical programming languages such as R, Python or automated scripts to filter the lists of bacterial species associated with specific human health conditions as shown in Fig. 6.

- A combination of standard microbial ecology measures (i.e., Alpha and Beta diversity) and the quantitative analysis was used to assess the impact of healthcare product on the human microbiome.

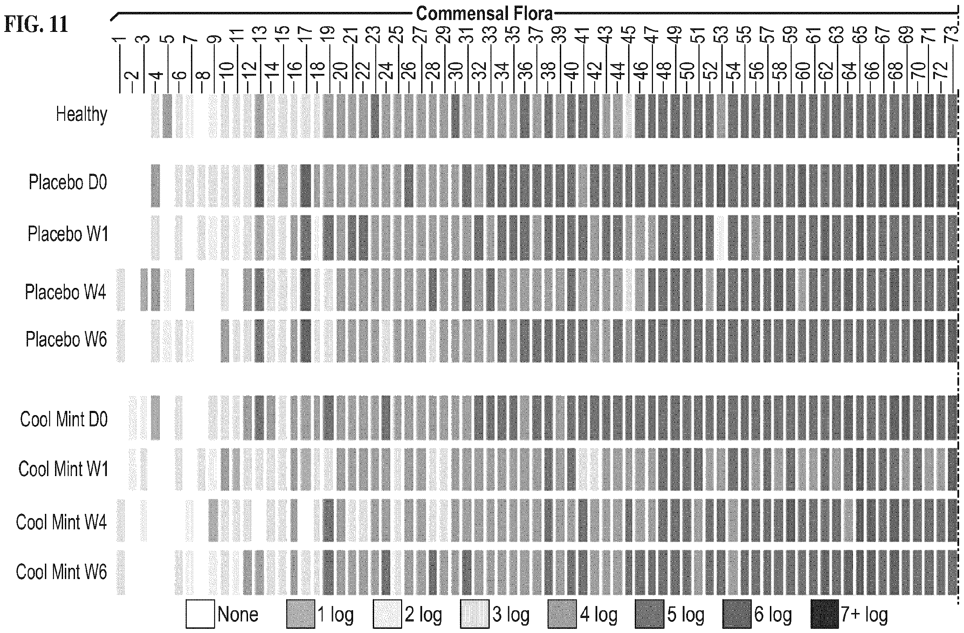

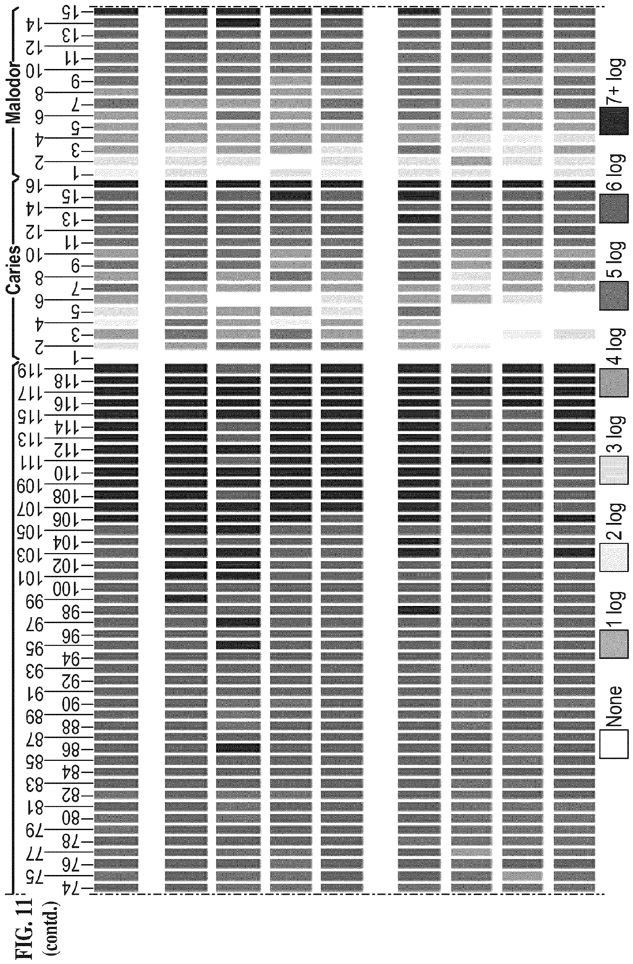

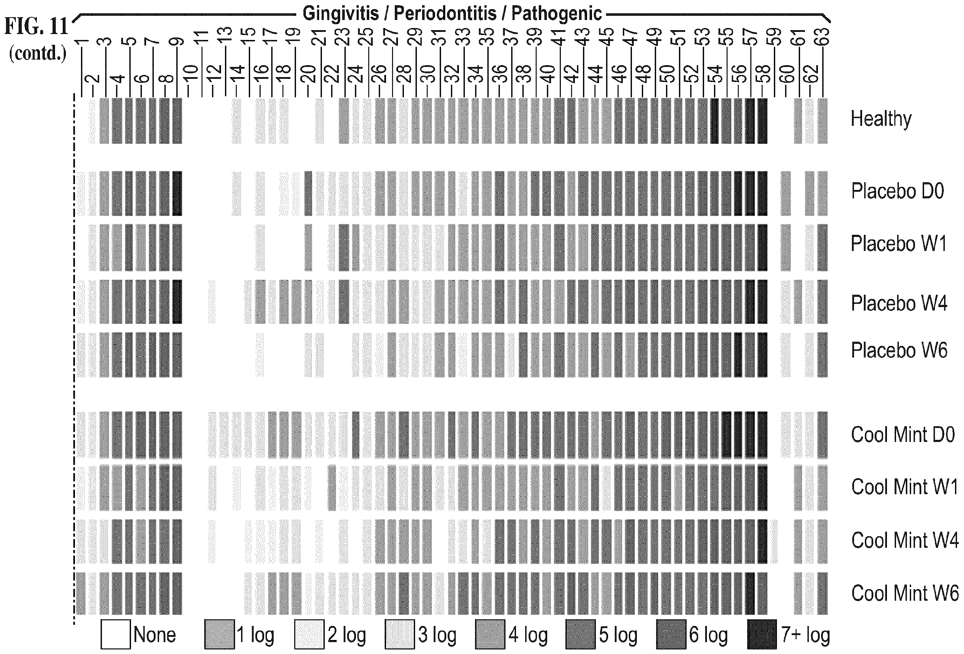

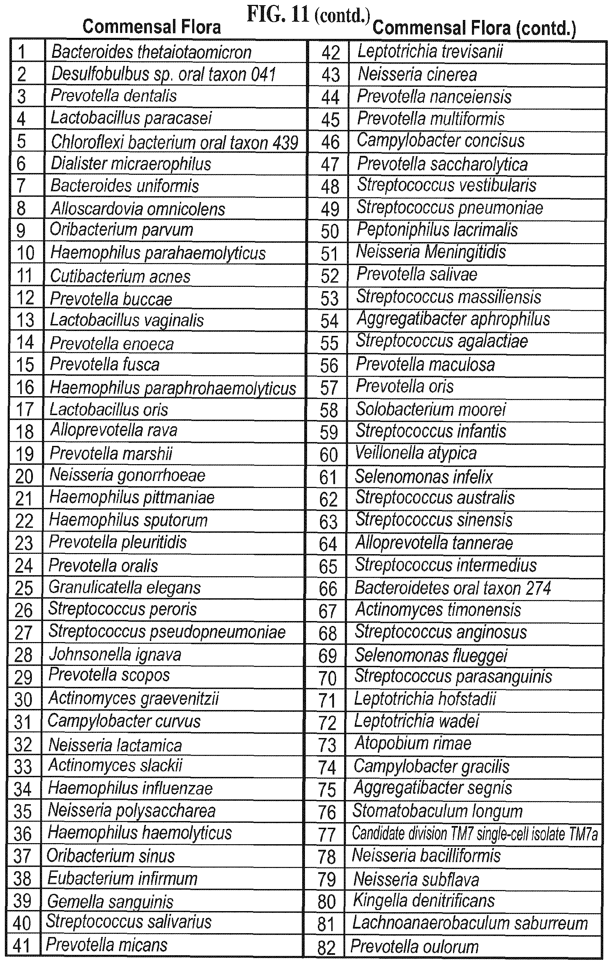

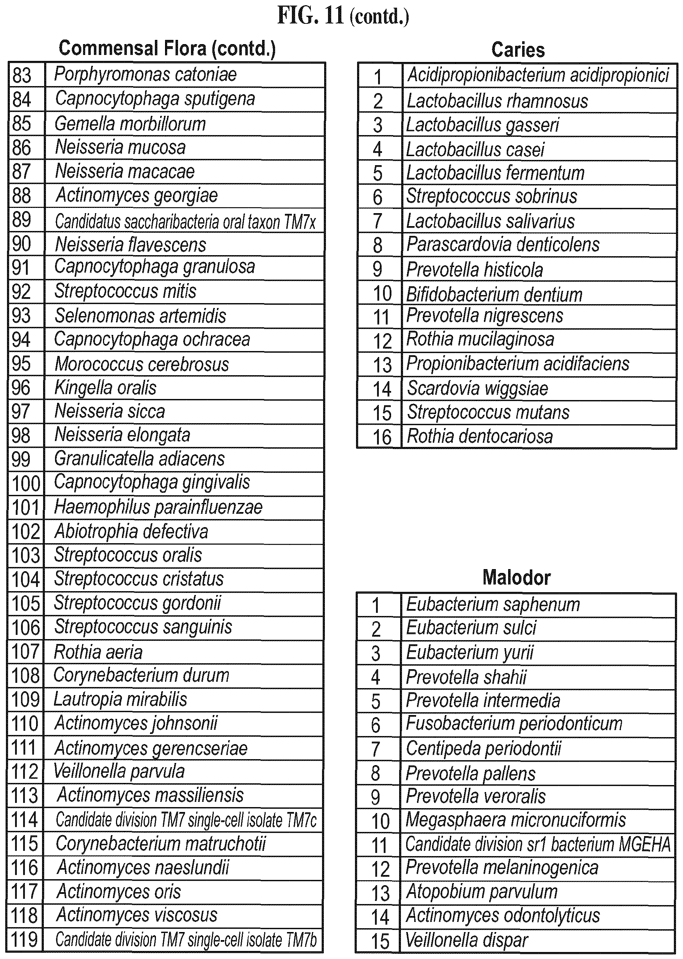

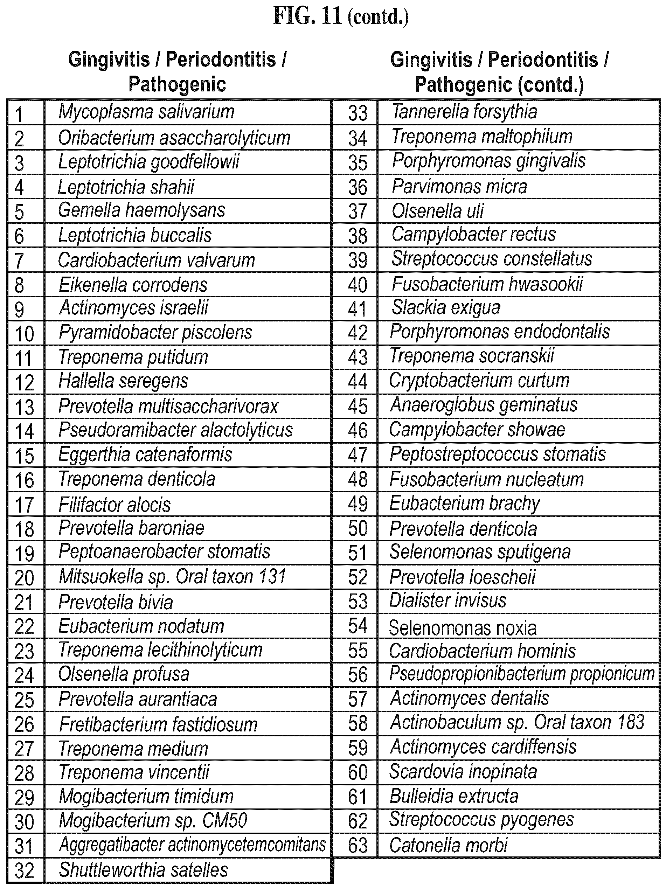

- For quantitative and meaningful assessment of product (e.g., consumer product) microbiome impact using data obtained from the above process steps of this example, the following criteria for data refinement were observed, namely the exclusion of (1) Unknown bacterial taxa with unassigned scientific names, (2) Transient Contaminants (or, as indicated above, bacteria not normally residing on humans or from other sites of human body not relevant to the study at hand) and (3) the spike-in control bacteria. Such assessment of microbiome impact was achieved by mapping the list of bacterial species in the abundance data of Fig. 11 and filtering out unwanted species of bacteria based on the data refinement criteria 1-3 outlined above.

- High level summarization of microbiome impact was achieved by comparing the Alpha diversity measures of observed richness and Shannon-Weaver diversity based on study design variables such as treatment types, visits, clinical outcome etc., followed by statistical significance testing using the analysis of variance (ANOVA) and post-hoc analysis using Tukey Honest Significant Difference test ($\alpha$=0.05) for simple study designs or mixed effects model using repeated measures for human clinical trials. Unlike conventional alpha diversity metrics obtained from 16S amplicon sequencing which has only limited sensitivity in taxonomic identification of bacteria to the species level, the combination of shotgun metagenome sequencing and data refinement described above provided a much more accurate summarization of microbiome.

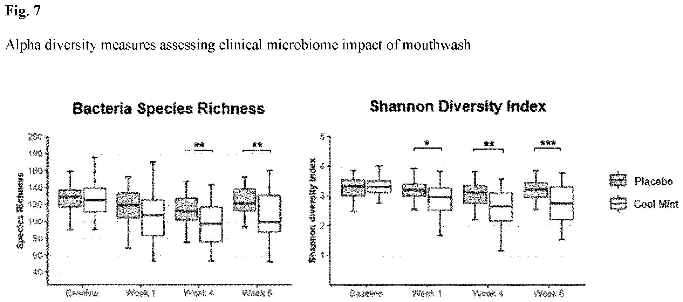

- Fig. 7 shows the alpha diversity measures assessing the clinical microbiome impact of mouthwash. Gingivitis subjects who used LISTERINE® Cool Mint Antiseptic Mouthwash showed a decrease in oral bacterial richness by approximately 20 species compared to placebo hydroalcohol after 6 weeks of twice daily use. The Shannon Diversity Index values also showed a decrease in overall oral microbiome diversity by approximately 0.8 index during this time. These statistically significant results demonstrate that the clinical improvements in gingivitis outcomes associated with LISTERINE® Cool Mint Antiseptic Mouthwash are supported by the mouthwash data showing reduced levels of bacterial species and diversity in the microbiome.

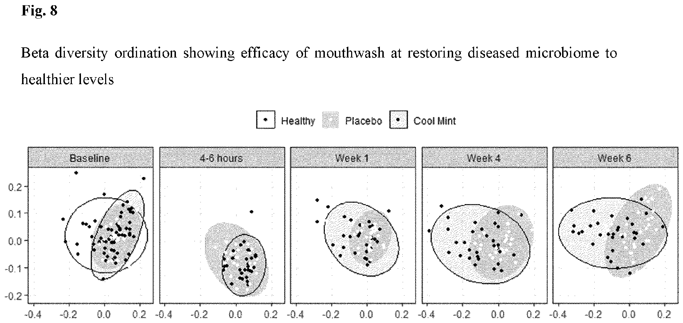

- Fig. 8 shows the beta diversity ordination illustrating the efficacy of the LISTERINE® Cool Mint Antiseptic Mouthwash at restoring diseased microbiome to healthier levels (i.e., less pathogenic bacterial species and reduced abundance of total bacteria). At baseline, a significant difference is observed in the microbiome composition of gingivitis subjects (as indicated by Placebo and LISTERINE® Cool Mint Antiseptic Mouthwash diagrams) compared to reference cohorts with naturally healthy mouth. Longitudinal twice daily use of LISTERINE® Cool Mint Antiseptic Mouthwash helps to return the microbiome composition of gingivitis subjects to a state similar to that of the naturally "healthy" reference cohorts by week 4.

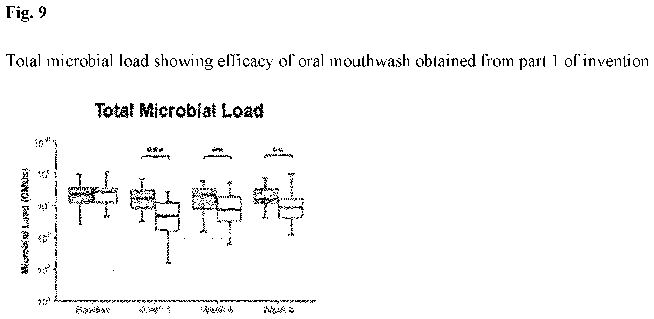

- Since alpha diversity measures are agnostic to bacterial cellular abundances, total microbial load can be assessed using data calculated as described in the above process steps of this example. This was achieved by calculating the aggregate sum abundances of all bacteria per each sample and comparing the mean CMU abundance values between the relevant groups of samples for comparison or the mean of log10 transformed abundance values. Fig. 9 shows reductions in the total microbial load (i.e., total number of bacteria in the plaque) of oral plaque microbiome after using LISTERINE® Cool Mint Antiseptic Mouthwash, indicating efficacy of such mouthwash.

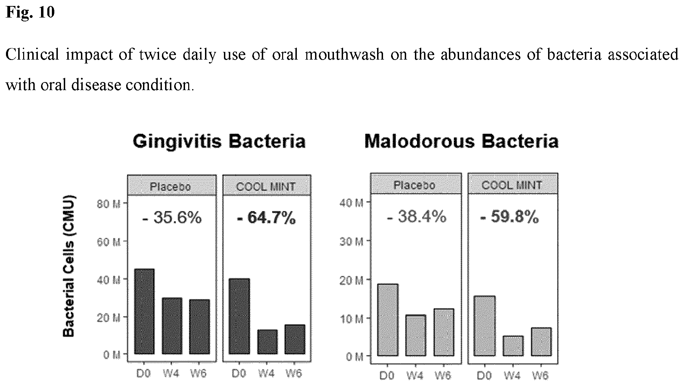

- To quantitatively assess a healthcare (e.g., oral care) product on specific groupings of bacteria associated with human health conditions, bacterial cellular abundances data obtained and bacterial species mapping metadata as obtained in the above process steps of this example were combined together and analyzed according to specific study design variables of interest. For example, the impact of a mouthwash formulation on gingivitis or malodor associated bacteria on supragingival plaque was assessed by aggregating the sum of abundances of all species associated with gingivitis (64 species) or malodor (29 species) per sample basis and comparing the group means of CMUs or the group means of log10 CMUs before vs after treatment and/or vs a placebo control. The results are shown in Fig. 10. The results show that after twice daily use for 4 weeks (W) and 6 weeks (W), gingivitis subjects using LISTERINE® Cool Mint Antiseptic Mouthwash maintained reduced amounts of all species of bacteria associated with gum disease and malodor by over 60% and close to 60%, respectively, as compared to the baseline (D). Cohorts randomized to the placebo (i.e., the brushing alone and hydroalcohol mouthwash control group), only showed reduction of gum disease and malodor causing bacterial species by less than 36% and less than 39%, respectively, only. Though commonly understood, the data demonstrates that, in fact, all species associated with gum disease and malodor is impacted by LISTERINE® Cool Mint Antiseptic Mouthwash.

- A detailed overview of the impact of healthcare products on oral microbiome bacterial species are shown in Figs. 10 and 11. The findings associated with the above process steps in this example are visually represented using "heatmaps" (employing distinct colors) by encoding the magnitude of bacterial cell numbers using the CMUs obtained from the above process steps of this example or their differences between product and placebo or before and after product treatment. The numerical values can be the average CMUs aggregated per product group and per visit or the average of Log10 CMUs.

- Assessment of individual bacterial abundances showed commensal species generally predominated the oral supragingival plaque in comparison to clinically relevant species associated with gum disease or malodor for both placebo subjects and LISTERINE® Cool Mint Antiseptic Mouthwash subjects (See Fig. 11).

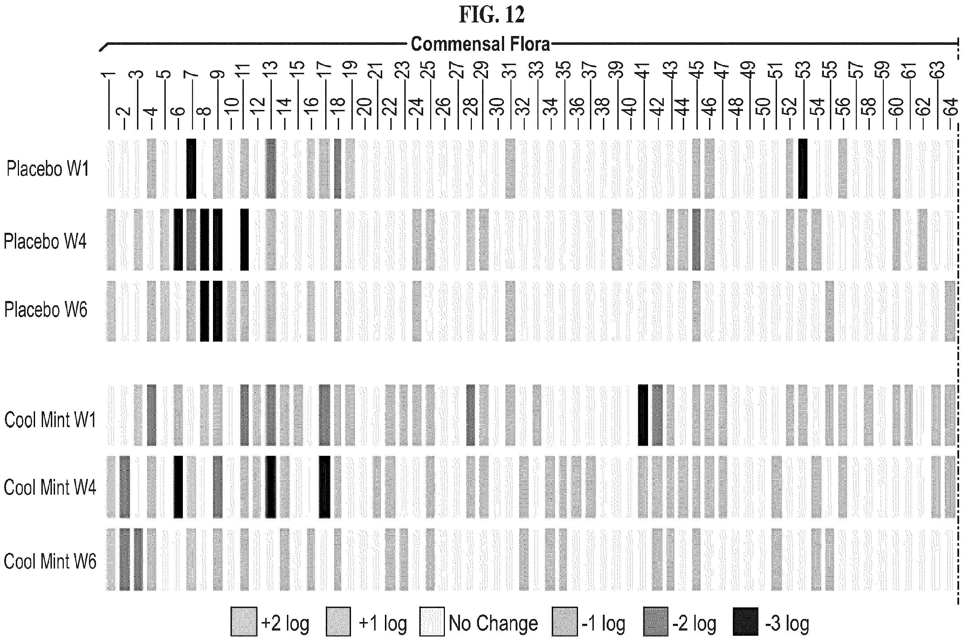

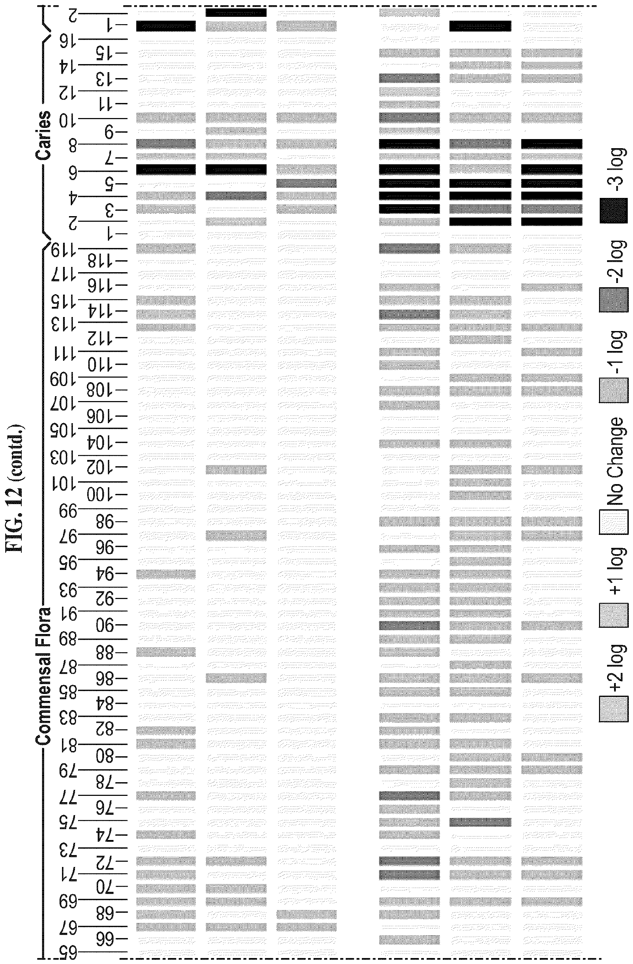

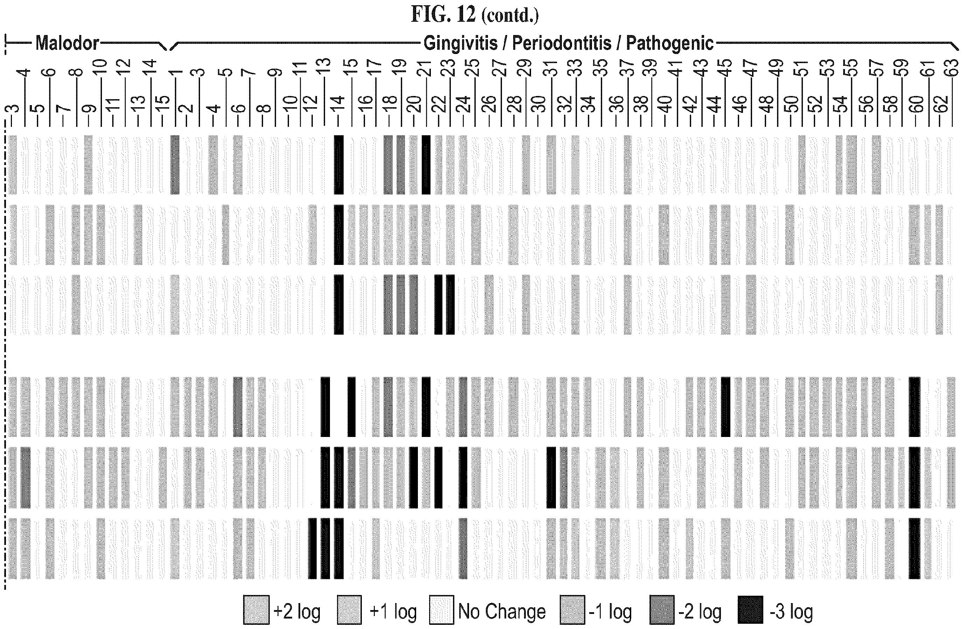

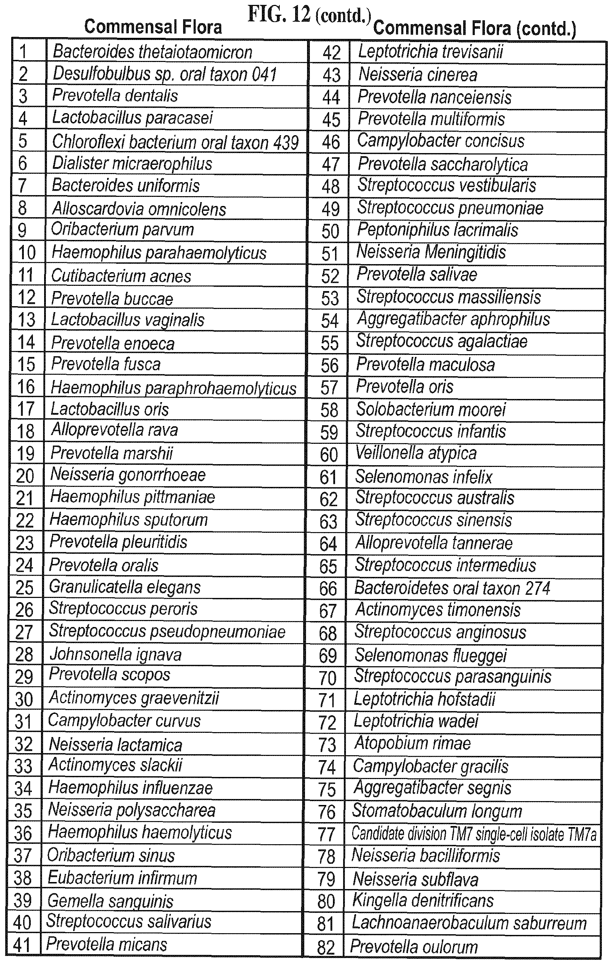

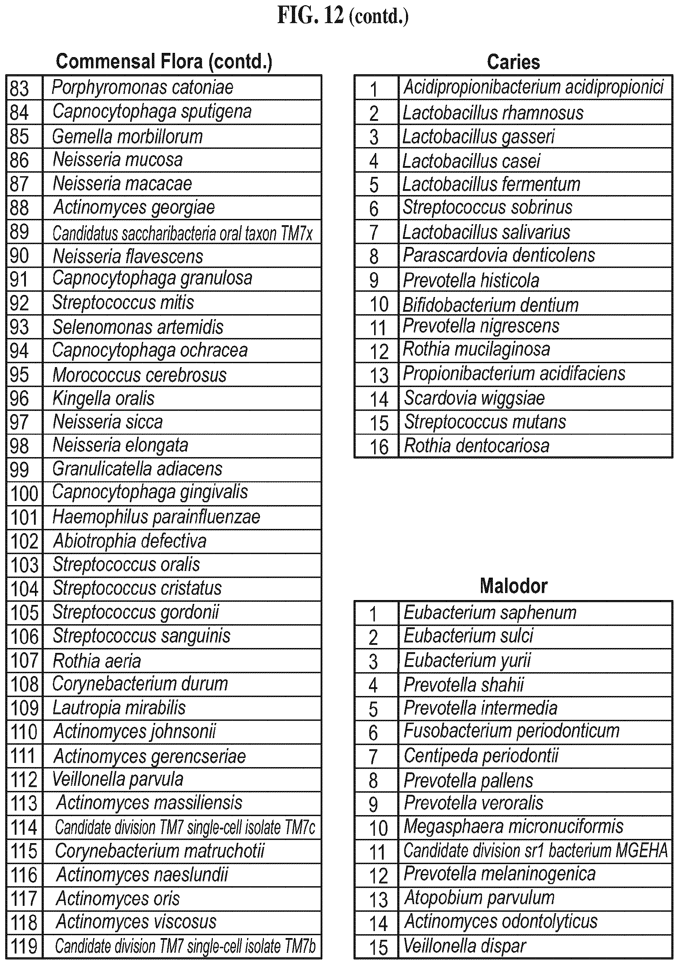

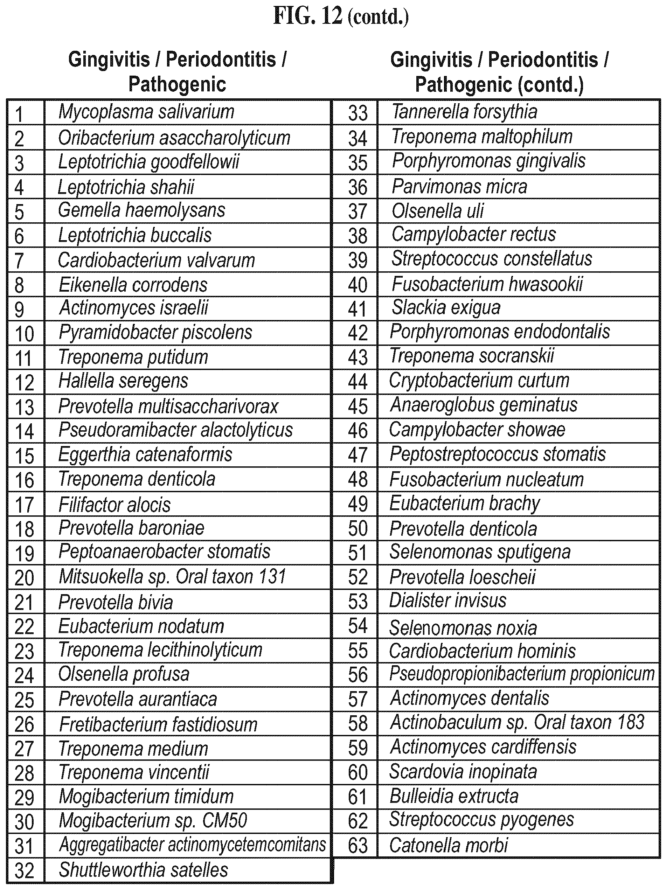

- After twice daily use of a mouthwash for 1 week, 4 weeks and 6 weeks, antimicrobial reductions of at least 1 logic or 90% compared to the baseline were observed for oral plaque bacteria species in gingivitis subjects using LISTERINE® Cool Mint Antiseptic Mouthwash but not in subjects using placebo hydroalcohol mouthwash (Fig. 12). The antimicrobial reductions observed were indiscriminate and did not selectively favor particular types of bacteria species over another.

Embodiments of the Present Invention:

1. A method for analyzing the microbiome of a human, mammal or non-human/non-mammal, comprising the steps of:

a. collecting at least one polymicrobial sample containing nucleic acid material (NA) from the human, mammal or non-human/non-mammal;

b. adding spike-in control standards to the sample of step a, which standard comprises at least two known microbial species not residing on/in the human, mammal or non-human/non-mammal, respectively, at known concentrations;

c. extracting NA from the microbial sample of step b;

d. analyzing the sample of step c using shotgun metagenome sequencing, wherein the technique provides comprehensive identification of the microbial organisms down to the species or strain taxonomic level and their abundances in the form of NA copy numbers or relative abundances;

e. calculate the cell numbers (or quantity or amount) for each of the microbial organisms identified in step d; and

f. mapping (or associating) on 1:n basis each microbial species identified in step d with human and/or mammal health conditions or causes of such health conditions based on known clinical testing information (including information obtained through calculation or extrapolation of relevant regional genomic or metabolomic measurements from a human and/or mammal) regarding the microbial species and the health conditions or causes of such health conditions.

2. The method of embodiment 1 and any of the following embodiments, wherein the step of the calculation of number (or quantity or amount) of the microbial organisms identified in step e comprises the steps of:

a. generating calibration curves of the control standards of step b for each sample against the NA copy numbers or relative abundances to calculate the amount or weight of genomic NA of microbial organisms;

b. determining the molecular weights of the microbial organisms identified at the species or strain taxonomic level from step d. using genome size information obtained from genome databases; and

c. calculate the cell numbers for each of the microbial organisms identified in step d using the following formula:

$$Cell\ number = \left(\frac{amount\ of\ genomic\ DNA\ in\ step\ e}{molecular\ weights\ determined\ in\ step\ f}\right) \times Avogadro's\ number.$$

3. The method of embodiments 1 and 2 and any of the following embodiments, comprising the step of determining the occurrence of either a change in microbial population of microbiome or no change in microbial population of microbiome.

4. The method of any of the preceding embodiments and any of the following embodiments, comprising the step of determining quantitative impact (i.e., causing microbial population reduction or growth) of one or more variables (including temperature, age, passing of time, environment (e.g., pollution), physical/mental stress, presence and/or absence of disease state, and use of a specific product or specific technology), and/or the time (or timing) of commencement (i.e., relative to the occurrence of intervention with such variables) of such impact on the cell number(s) of one or more microbial species of the human and/or mammal microbiome by combining and organizing the cell number data from step e with the mapping data obtained from step f.

5. The method of any of the preceding embodiments and any of the following embodiments, wherein the impact is a clinically relevant impact.

6. The method of any of the preceding embodiments and any of the following embodiments, comprising the step of excluding from the method any microbial taxa which have not been isolated for taxonomic classification and/or transient microbial species not normally residing on/in regions of interest on the bodies of humans, mammals or non-human/non-mammal sources.

7. The method of any of embodiments 1-5 and any of the following embodiments, further comprising the step of excluding from the method any microbial taxa which have been previously isolated (and/or are known in the art as residing in the microbiome of interest) for taxonomic classification and/or transient microbial species which are normally residing on/in regions of interest on the bodies of humans, mammals or non-human/non-mammal sources, for facilitating a more focused assessment of unknown or transient microbial taxa that may reside in the microbiome of interest.

8. A method for analyzing the microbiome of a human, mammal or non-human/non-mammal, comprising the steps of:

a. providing a sample collection kit for a user to collect at least one polymicrobial sample containing NA from the human, mammal or non-human/non-mammal;

b. adding spike-in control standards to the sample of step a, which standard comprises at least two known microbial species not residing on/in the human, mammal or non-human/non-mammal, respectively at known concentrations;

c. extracting NA from the microbial sample of step b;

d. analyzing and the sample of step c using shotgun metagenome sequencing, wherein the shotgun provides comprehensive identification of the microbial organisms down to the species or strain taxonomic level and their abundances in the form of NA copy numbers or relative abundances;

e. calculate the cell numbers (or quantity or amount) for each of the microbial organisms identified in step d; and

f. mapping (or associating) on 1:n basis each microbial species identified in step d with human and/or mammal health conditions or causes of such health conditions based on known clinical testing information (including information obtained through calculation or extrapolation of relevant regional genomic or metabolomic measurements from a human and/or mammal) regarding the microbial species and the health conditions or causes of such health conditions;

g. obtaining current and historical data (e.g., health data) on the user related to the health conditions or causes of such health conditions of step f;

h. determining a user or source reference population, the determination comprising the steps of:

> i. identifying a plurality of human individuals, mammals or non-human/non-mammal sources having at least one common characteristic (such as reside in the same geographic region, included in same population age group, have same gender, practice same living habits);
>
> ii. collecting at least one polymicrobial sample containing NA from the human individuals, mammals or non-human/non-mammal sources in the user or source reference population; and
>
> iii. repeating steps b through g for each individual, mammal or non-human/non-mammal source in the user or source reference population;
>
> iv. determining the quantitative impact of a product or technology on the health condition or causes of such health condition of healthy human individuals in the user or source reference population related to the trait; and

> i. comparing the data from step f. for the user with the data of step h. for the user or source reference population.

9. The method of embodiment 8, any of the preceding embodiments and any of the following embodiments, comprising the step of advising the user on potential efficacy of the product or technology for the health condition or causes of such health condition.

10. A method for screening a compound and/or formulation for impact on a microbiome of a human, mammal or non-human/non-mammal source comprising the steps of:

> a. analyzing the microbiome of the human, mammal or non-human/non-mammal source, comprising the steps of:

>> i) collecting at least one polymicrobial sample containing NA from a bodily region of interest on the human or mammal or from the non-human/non-mammal source wherein the sample is a first sample;
>>
>> ii) adding spike-in control standards to the sample of step a, which standard comprises at least two known microbial species not residing on/in the human, mammal or non-human/non-mammal, respectively at known concentrations;
>>
>> iii) extracting NA from the microbial sample of step b;
>>
>> iv) analyzing and the sample of step c using shotgun metagenome sequencing, wherein the shotgun provides comprehensive identification of the microbial organisms down to the species or strain taxonomic level and their abundances in the form of NA copy numbers or relative abundances;
>>
>> v) calculate the cell numbers (or quantity or amount) for each of the microbial organisms identified in step d; and
>>
>> vi) mapping (or associating) on 1:n basis each microbial species identified in step d with human and/or mammal health conditions or causes of such health conditions based on known clinical testing information (including information obtained through calculation or extrapolation of relevant regional genomic or metabolomic measurements from a human and/or mammal) regarding the microbial species and the health condition or causes of such health condition;

b. analyzing the impact of a first formulation or compound on the microbiome of the human, mammal or non-human/non-mammal source of step a) comprising:

A. administering the first formulation or compound to the bodily region of interest on the human or mammal or to the non-human/non-mammal source;
B. repeating the steps a.(i) through a.(vi) for a second at least one polymicrobial sample obtained from the bodily region of interest on the human, mammal or non-human/non-mammal source after administering the first formulation or compound; and

c. comparing the data from step a.(vi) for the first sample with the data of step b. obtained upon completing the repeat of step a.(vi) for the second sample.

11. The method of embodiment 10, any of the preceding embodiments and any of the following embodiments, further comprising the steps of:

a. analyzing the impact of a second formulation or compound on the microbiome of the human or mammal of step claim 10a. comprising:

A. administering the second formulation or compound to a bodily region of interest on the human or mammal or to the non-human/non-mammal source,
B. repeating the steps 1 0a. (i) through a.(vi) for a third at least one polymicrobial sample obtained from the bodily region of interest on the human, mammal or non-human/non-mammal source after administering the first formulation or compound; and

b. comparing the data obtained from step claim 11a.B, upon completing the repeat of step 10a. (vi)for the third sample, with:

A. the data of step claim 10a.(vi) for the first sample; and/or
B. the data of step claim 10b. for the second sample, obtained upon completing the repeat of step claim 10a. (vi) for the second sample.

12. The method of embodiment 10, any of the preceding embodiments and any of the following embodiments, wherein the first sample forms a baseline microbiome measurement.

13. The method of embodiment 10, any of the preceding embodiments and any of the following embodiments, wherein the second sample forms an experimental microbiome measurement.

14. The method of embodiment 11 and any of the preceding embodiments, wherein the third sample forms a benchmark microbiome measurement

**Claims**

1. A method for analyzing the microbiome of a human, mammal or non-human/non-mammal, comprising the steps of:

a. collecting at least one polymicrobial sample containing nucleic acid material (NA) from the human, mammal or non-human/non-mammal;
b. adding spike-in control standards to the sample of step a, which standard comprises at least two known microbial species not residing on/in the human, mammal or non-human/non-mammal, respectively, at known concentrations;
c. extracting NA from the microbial sample of step b;
d. analyzing the sample of step c using shotgun metagenome sequencing, wherein the technique provides comprehensive identification of the microbial organisms down to the species or strain taxonomic level and their abundances in the form of NA copy numbers or relative abundances;
e. calculate the cell numbers for each of the microbial organisms identified in step d; and
f. mapping on 1:n basis each microbial species identified in step d with human and/or mammal health conditions or causes of such health conditions based on known clinical testing information regarding the microbial species and the health conditions or causes of such health conditions.

**2.** The method of claim 1 and any of the following embodiments, wherein the step of the calculation of number of the microbial organisms identified in step e comprises the steps of:

a. generating calibration curves of the control standards of step b for each sample against the NA copy numbers or relative abundances to calculate the amount or weight of genomic NA of microbial organisms;
b. determining the molecular weights of the microbial organisms identified at the species or strain taxonomic level from step d. using genome size information obtained from genome databases; and
c. calculate the cell numbers for each of the microbial organisms identified in step d using the following formula:

$$Cell\ number = \left(\frac{amount\ of\ genomic\ DNA\ in\ step\ e}{molecular\ weights\ determined\ in\ step\ f}\right) \times Avogadro's\ number.$$

**3.** The method of claim 1, comprising the step of determining the occurrence of either a change in microbial population of microbiome or no change in microbial population of microbiome.

**4.** The method of claim 1, comprising the step of determining quantitative impact of one or more variables, and/or the time of commencement of such impact on the cell number(s) of one or more microbial species of the human and/or mammal microbiome by combining and organizing the cell number data from step e with the mapping data obtained from step f.

**5.** The method of claim 4, wherein the impact is a clinically relevant impact.

**6.** The method of claim 1, comprising the step of excluding from the method any microbial taxa which have not been isolated for taxonomic classification and/or transient microbial species not normally residing on/in regions of interest on the bodies of humans, mammals or non-human/non-mammal sources.

**7.** The method of claim 1, further comprising the step of excluding from the method any microbial taxa which have been previously isolated for taxonomic classification and/or transient microbial species which are normally residing on/in regions of interest on the bodies of humans, mammals or non-human/non-mammal sources.

**8.** A method for analyzing the microbiome of a human, mammal or non-human/non-mammal, comprising the steps of:

a. providing a sample collection kit for a user to collect at least one polymicrobial sample containing NA from the human, mammal or non-human/non-mammal;
b. adding spike-in control standards to the sample of step a, which standard comprises at least two known microbial species not residing on/in the human, mammal or non-human/non-mammal, respectively at known concentrations;
c. extracting NA from the microbial sample of step b;
d. analyzing and the sample of step c using shotgun metagenome sequencing, wherein the shotgun provides comprehensive identification of the microbial organisms down to the species or strain taxonomic level and their abundances in the form of NA copy numbers or relative abundances;
e. calculate the cell numbers for each of the microbial organisms identified in step d; and
f. mapping on 1:n basis each microbial species identified in step d with human and/or mammal health conditions or causes of such health conditions based on known clinical testing information regarding the microbial species and the health conditions or causes of such health conditions;
g. obtaining current and historical data on the user related to the health conditions or causes of such health conditions of step f;
h. determining a user or source reference population, the determination comprising the steps of:

i. identifying a plurality of human individuals, mammals or non-human/non-mammal sources having at least one common characteristic (such as reside in the same geographic region, included in same population age group, have same gender, practice same living habits);
ii. collecting at least one polymicrobial sample containing NA from the human individuals, mammals or non-human/non-mammal sources in the user or source reference population; and
iii. repeating steps b through g for each individual, mammal or non-human/non-mammal source in the user or source reference population;
iv. determining the quantitative impact of a product or technology on the health condition or causes of such health condition of healthy human individuals in the user or source reference population related to the trait; and

i. comparing the data from step f. for the user with the data of step h. for the user or source reference population.

9. The method of claim 8, comprising the step of advising the user on potential efficacy of the product or technology for the health condition or causes of such health condition.

10. A method for screening a compound and/or formulation for impact on a microbiome of a human, mammal or non-human/non-mammal source comprising the steps of:

a. analyzing the microbiome of the human, mammal or non-human/non-mammal source, comprising the steps of:

i) collecting at least one polymicrobial sample containing NA from a bodily region of interest on the human or mammal or from the non-human/non-mammal source wherein the sample is a first sample;
ii) adding spike-in control standards to the sample of step a, which standard comprises at least two known microbial species not residing on/in the human, mammal or non-human/non-mammal, respectively at known concentrations;
iii) extracting NA from the microbial sample of step b;
iv) analyzing and the sample of step c using shotgun metagenome sequencing, wherein the shotgun provides comprehensive identification of the microbial organisms down to the species or strain taxonomic level and their abundances in the form of NA copy numbers or relative abundances;
v) calculate the cell numbers for each of the microbial organisms identified in step d; and
vi) mapping on 1:n basis each microbial species identified in step d with human and/or mammal health conditions or causes of such health conditions based on known clinical testing information regarding the microbial species and the health condition or causes of such health condition;

b. analyzing the impact of a first formulation or compound on the microbiome of the human, mammal or non-human/non-mammal source of step a) comprising:

A. administering the first formulation or compound to the bodily region of interest on the human or mammal or to the non-human/non-mammal source;
B. repeating the steps a.(i) through a.(vi) for a second at least one polymicrobial sample obtained from the bodily region of interest on the human, mammal or non-human/non-mammal source after administering the first formulation or compound; and

c. comparing the data from step a.(vi) for the first sample with the data of step b. obtained upon completing the repeat of step a.(vi) for the second sample.

11. The method of claim 10, comprising the steps of:

a. analyzing the impact of a second formulation or compound on the microbiome of the human or mammal of step claim 10a. comprising:

A. administering the second formulation or compound to a bodily region of interest on the human or mammal or to the non-human/non-mammal source,
B. repeating the steps 10a. (i) through a.(vi) for a third at least one polymicrobial sample obtained from the bodily region of interest on the human, mammal or non-human/non-mammal source after administering the first formulation or compound; and

b. comparing the data obtained from step claim 11a.B, upon completing the repeat of step 10a. (vi) for the third sample, with:

A. the data of step claim 10a.(vi) for the first sample; and/or
B. the data of step claim 10b. for the second sample, obtained upon completing the repeat of step claim 10a. (vi) for the second sample.

12. The method of claim 10, wherein the first sample forms a baseline microbiome measurement.

**13.** The method of claim 10, wherein the second sample forms an experimental microbiome measurement.

**14.** The method of claim 11, wherein the third sample forms a benchmark microbiome measurement

Fig. 1

Comparison of microbiome relative abundance *vs* absolute abundance quantification

**Fig. 2**

Three parts impact quantifying method of present invention

**Fig. 3**

Standard calibration curve (output abundance vs input DNA amount)

$$y = 43.227x + 0.0653$$
$$R^2 = 0.9993$$

**Fig. 4**

Data structure for bacterial species mapping to health conditions

| Index | Variables | Values |
|---|---|---|
| Bacterial species | Cariogenic or Acidogenic | Yes or No or Blank |
| Bacterial strains | Gingivitis | Yes or No or Blank |
| | Periodontitis | Yes or No or Blank |
| | Malodor | Yes or No or Blank |
| | Commensal | Yes or No or Blank |
| | Pathogenic | Yes or No or Blank |
| | Healthy | Yes or No or Blank |
| | Unknown | Yes or No or Blank |
| | Contaminants | Yes or No or Blank |
| | Aerobic | Yes or No or Blank |
| | Anaerobic | Yes or No or Blank |
| | Etc.. | Etc.. |

**Fig. 5**

Bacterial species mapping metadata for oral care application

| Name | Cavities | Gingivitis | Periodont | Malodor | Commens | Pathogen | Healthy | Unknown | Contamin | Source |
|---|---|---|---|---|---|---|---|---|---|---|
| [Eubacterium] brachy | No | Yes | Yes | No | No | Yes | No | No | No | Oral |
| [Eubacterium] infirmum | No | No | No | No | Yes | No | No | No | No | Oral |
| [Eubacterium] nodatum | No | Yes | Yes | Yes | No | Yes | No | No | No | Oral |
| [Eubacterium] rectale | | | | | | | | | Yes | Feces |
| [Eubacterium] saphenum | No | Yes | Yes | Yes | No | No | No | No | No | Oral |
| [Eubacterium] sulci | | Yes | Yes | Yes | | | | No | No | Oral |
| [Eubacterium] yurii | | | Yes | Yes | | | | No | No | Oral |
| [Hallella] seregens | | Yes | Yes | | | | | No | No | Oral |

**Fig. 6**

Bacterial species classification derived from mapping metadata for oral care

| | Cariogenic | Gingivitis | Malodor |
|---|---|---|---|
| 1 | | | |
| 2 | Bifidobacterium dentium | Actinobaculum sp. oral taxon 183 | Actinomyces odontolyticus |
| 3 | Catonella morbi | Actinomyces dentalis | Atopobium parvulum |
| 4 | Dialister invisus | Actinomyces israelii | candidate division SR1 bacterium MGEHA |
| 5 | Eggerthia catenaformis | Aggregatibacter actinomycetemcomitans | Centipeda periodontii |
| 6 | Lactobacillus casei | Anaeroglobus geminatus | Cryptobacterium curtum |
| 7 | Lactobacillus fermentum | Campylobacter rectus | Dialister invisus |
| 8 | Lactobacillus gasseri | Campylobacter showae | Eubacterium nodatum |
| 9 | Lactobacillus rhamnosus | Cardiobacterium hominis | Eubacterium saphenum |
| 10 | Lactobacillus salivarius | Cardiobacterium valvarum | Eubacterium sulci |
| 11 | Olsenella profusa | Centipeda periodontii | Eubacterium yurii |
| 12 | Parascardovia denticolens | Cryptobacterium curtum | Fusobacterium nucleatum |
| 13 | Prevotella histicola | Dialister invisus | Fusobacterium periodonticum |
| 14 | Prevotella nigrescens | Eggerthia catenaformis | Megasphaera micronuciformis |
| 15 | Propionibacterium acidifaciens | Eikenella corrodens | Neisseria flavescens |
| 16 | Rothia dentocariosa | Eubacterium brachy | Porphyromonas endodontalis |
| 17 | Rothia mucilaginosa | Eubacterium nodatum | Porphyromonas gingivalis |
| 18 | Scardovia inopinata | Eubacterium saphenum | Prevotella intermedia |
| 19 | Scardovia wiggsiae | Eubacterium sulci | Prevotella loescheii |
| 20 | Streptococcus mutans | Eubacterium yurii | Prevotella melaninogenica |
| 21 | Streptococcus sobrinus | Filifactor alocis | Prevotella pallens |

**Fig. 7**

Alpha diversity measures assessing clinical microbiome impact of mouthwash

**Fig. 8**

Beta diversity ordination showing efficacy of mouthwash at restoring diseased microbiome to healthier levels

**Fig. 9**

Total microbial load showing efficacy of oral mouthwash obtained from part 1 of invention

**Fig. 10**

Clinical impact of twice daily use of oral mouthwash on the abundances of bacteria associated with oral disease condition.

FIG. 11

FIG. 11 (contd.)

FIG. 11
(contd.)

## FIG. 11 (contd.)

| Commensal Flora | | | Commensal Flora (contd.) | |
|---|---|---|---|---|
| 1 | Bacteroides thetaiotaomicron | | 42 | Leptotrichia trevisanii |
| 2 | Desulfobulbus sp. oral taxon 041 | | 43 | Neisseria cinerea |
| 3 | Prevotella dentalis | | 44 | Prevotella nanceiensis |
| 4 | Lactobacillus paracasei | | 45 | Prevotella multiformis |
| 5 | Chloroflexi bacterium oral taxon 439 | | 46 | Campylobacter concisus |
| 6 | Dialister micraerophilus | | 47 | Prevotella saccharolytica |
| 7 | Bacteroides uniformis | | 48 | Streptococcus vestibularis |
| 8 | Alloscardovia omnicolens | | 49 | Streptococcus pneumoniae |
| 9 | Oribacterium parvum | | 50 | Peptoniphilus lacrimalis |
| 10 | Haemophilus parahaemolyticus | | 51 | Neisseria Meningitidis |
| 11 | Cutibacterium acnes | | 52 | Prevotella salivae |
| 12 | Prevotella buccae | | 53 | Streptococcus massiliensis |
| 13 | Lactobacillus vaginalis | | 54 | Aggregatibacter aphrophilus |
| 14 | Prevotella enoeca | | 55 | Streptococcus agalactiae |
| 15 | Prevotella fusca | | 56 | Prevotella maculosa |
| 16 | Haemophilus paraphrohaemolyticus | | 57 | Prevotella oris |
| 17 | Lactobacillus oris | | 58 | Solobacterium moorei |
| 18 | Alloprevotella rava | | 59 | Streptococcus infantis |
| 19 | Prevotella marshii | | 60 | Veillonella atypica |
| 20 | Neisseria gonorrhoeae | | 61 | Selenomonas infelix |
| 21 | Haemophilus pittmaniae | | 62 | Streptococcus australis |
| 22 | Haemophilus sputorum | | 63 | Streptococcus sinensis |
| 23 | Prevotella pleuritidis | | 64 | Alloprevotella tannerae |
| 24 | Prevotella oralis | | 65 | Streptococcus intermedius |
| 25 | Granulicatella elegans | | 66 | Bacteroidetes oral taxon 274 |
| 26 | Streptococcus peroris | | 67 | Actinomyces timonensis |
| 27 | Streptococcus pseudopneumoniae | | 68 | Streptococcus anginosus |
| 28 | Johnsonella ignava | | 69 | Selenomonas flueggei |
| 29 | Prevotella scopos | | 70 | Streptococcus parasanguinis |
| 30 | Actinomyces graevenitzii | | 71 | Leptotrichia hofstadii |
| 31 | Campylobacter curvus | | 72 | Leptotrichia wadei |
| 32 | Neisseria lactamica | | 73 | Atopobium rimae |
| 33 | Actinomyces slackii | | 74 | Campylobacter gracilis |
| 34 | Haemophilus influenzae | | 75 | Aggregatibacter segnis |
| 35 | Neisseria polysaccharea | | 76 | Stomatobaculum longum |
| 36 | Haemophilus haemolyticus | | 77 | Candidate division TM7 single-cell isolate TM7a |
| 37 | Oribacterium sinus | | 78 | Neisseria bacilliformis |
| 38 | Eubacterium infirmum | | 79 | Neisseria subflava |
| 39 | Gemella sanguinis | | 80 | Kingella denitrificans |
| 40 | Streptococcus salivarius | | 81 | Lachnoanaerobaculum saburreum |
| 41 | Prevotella micans | | 82 | Prevotella oulorum |

## FIG. 11 (contd.)

### Commensal Flora (contd.)

| 83 | Porphyromonas catoniae |
|---|---|
| 84 | Capnocytophaga sputigena |
| 85 | Gemella morbillorum |
| 86 | Neisseria mucosa |
| 87 | Neisseria macacae |
| 88 | Actinomyces georgiae |
| 89 | Candidatus saccharibacteria oral taxon TM7x |
| 90 | Neisseria flavescens |
| 91 | Capnocytophaga granulosa |
| 92 | Streptococcus mitis |
| 93 | Selenomonas artemidis |
| 94 | Capnocytophaga ochracea |
| 95 | Morococcus cerebrosus |
| 96 | Kingella oralis |
| 97 | Neisseria sicca |
| 98 | Neisseria elongata |
| 99 | Granulicatella adiacens |
| 100 | Capnocytophaga gingivalis |
| 101 | Haemophilus parainfluenzae |
| 102 | Abiotrophia defectiva |
| 103 | Streptococcus oralis |
| 104 | Streptococcus cristatus |
| 105 | Streptococcus gordonii |
| 106 | Streptococcus sanguinis |
| 107 | Rothia aeria |
| 108 | Corynebacterium durum |
| 109 | Lautropia mirabilis |
| 110 | Actinomyces johnsonii |
| 111 | Actinomyces gerencseriae |
| 112 | Veillonella parvula |
| 113 | Actinomyces massiliensis |
| 114 | Candidate division TM7 single-cell isolate TM7c |
| 115 | Corynebacterium matruchotii |
| 116 | Actinomyces naeslundii |
| 117 | Actinomyces oris |
| 118 | Actinomyces viscosus |
| 119 | Candidate division TM7 single-cell isolate TM7b |

### Caries

| 1 | Acidipropionibacterium acidipropionici |
|---|---|
| 2 | Lactobacillus rhamnosus |
| 3 | Lactobacillus gasseri |
| 4 | Lactobacillus casei |
| 5 | Lactobacillus fermentum |
| 6 | Streptococcus sobrinus |
| 7 | Lactobacillus salivarius |
| 8 | Parascardovia denticolens |
| 9 | Prevotella histicola |
| 10 | Bifidobacterium dentium |
| 11 | Prevotella nigrescens |
| 12 | Rothia mucilaginosa |
| 13 | Propionibacterium acidifaciens |
| 14 | Scardovia wiggsiae |
| 15 | Streptococcus mutans |
| 16 | Rothia dentocariosa |

### Malodor

| 1 | Eubacterium saphenum |
|---|---|
| 2 | Eubacterium sulci |
| 3 | Eubacterium yurii |
| 4 | Prevotella shahii |
| 5 | Prevotella intermedia |
| 6 | Fusobacterium periodonticum |
| 7 | Centipeda periodontii |
| 8 | Prevotella pallens |
| 9 | Prevotella veroralis |
| 10 | Megasphaera micronuciformis |
| 11 | Candidate division sr1 bacterium MGEHA |
| 12 | Prevotella melaninogenica |
| 13 | Atopobium parvulum |
| 14 | Actinomyces odontolyticus |
| 15 | Veillonella dispar |

# FIG. 11 (contd.)

| Gingivitis / Periodontitis / Pathogenic | | Gingivitis / Periodontitis / Pathogenic (contd.) | |
|---|---|---|---|
| 1 | Mycoplasma salivarium | 33 | Tannerella forsythia |
| 2 | Oribacterium asaccharolyticum | 34 | Treponema maltophilum |
| 3 | Leptotrichia goodfellowii | 35 | Porphyromonas gingivalis |
| 4 | Leptotrichia shahii | 36 | Parvimonas micra |
| 5 | Gemella haemolysans | 37 | Olsenella uli |
| 6 | Leptotrichia buccalis | 38 | Campylobacter rectus |
| 7 | Cardiobacterium valvarum | 39 | Streptococcus constellatus |
| 8 | Eikenella corrodens | 40 | Fusobacterium hwasookii |
| 9 | Actinomyces israelii | 41 | Slackia exigua |
| 10 | Pyramidobacter piscolens | 42 | Porphyromonas endodontalis |
| 11 | Treponema putidum | 43 | Treponema socranskii |
| 12 | Hallella seregens | 44 | Cryptobacterium curtum |
| 13 | Prevotella multisaccharivorax | 45 | Anaeroglobus geminatus |
| 14 | Pseudoramibacter alactolyticus | 46 | Campylobacter showae |
| 15 | Eggerthia catenaformis | 47 | Peptostreptococcus stomatis |
| 16 | Treponema denticola | 48 | Fusobacterium nucleatum |
| 17 | Filifactor alocis | 49 | Eubacterium brachy |
| 18 | Prevotella baroniae | 50 | Prevotella denticola |
| 19 | Peptoanaerobacter stomatis | 51 | Selenomonas sputigena |
| 20 | Mitsuokella sp. Oral taxon 131 | 52 | Prevotella loescheii |
| 21 | Prevotella bivia | 53 | Dialister invisus |
| 22 | Eubacterium nodatum | 54 | Selenomonas noxia |
| 23 | Treponema lecithinolyticum | 55 | Cardiobacterium hominis |
| 24 | Olsenella profusa | 56 | Pseudopropionibacterium propionicum |
| 25 | Prevotella aurantiaca | 57 | Actinomyces dentalis |
| 26 | Fretibacterium fastidiosum | 58 | Actinobaculum sp. Oral taxon 183 |
| 27 | Treponema medium | 59 | Actinomyces cardiffensis |
| 28 | Treponema vincentii | 60 | Scardovia inopinata |
| 29 | Mogibacterium timidum | 61 | Bulleidia extructa |
| 30 | Mogibacterium sp. CM50 | 62 | Streptococcus pyogenes |
| 31 | Aggregatibacter actinomycetemcomitans | 63 | Catonella morbi |
| 32 | Shuttleworthia satelles | | |

FIG. 12

+2 log    +1 log    No Change    -1 log    -2 log    -3 log

FIG. 12 (contd.)

FIG. 12 (contd.)

Malodor — Gingivitis / Periodontitis / Pathogenic

-3 log
-2 log
-1 log
No Change
+1 log
+2 log

## FIG. 12 (contd.)

**Commensal Flora**               **Commensal Flora (contd.)**

| | | | |
|---|---|---|---|
| 1 | Bacteroides thetaiotaomicron | 42 | Leptotrichia trevisanii |
| 2 | Desulfobulbus sp. oral taxon 041 | 43 | Neisseria cinerea |
| 3 | Prevotella dentalis | 44 | Prevotella nanceiensis |
| 4 | Lactobacillus paracasei | 45 | Prevotella multiformis |
| 5 | Chloroflexi bacterium oral taxon 439 | 46 | Campylobacter concisus |
| 6 | Dialister micraerophilus | 47 | Prevotella saccharolytica |
| 7 | Bacteroides uniformis | 48 | Streptococcus vestibularis |
| 8 | Alloscardovia omnicolens | 49 | Streptococcus pneumoniae |
| 9 | Oribacterium parvum | 50 | Peptoniphilus lacrimalis |
| 10 | Haemophilus parahaemolyticus | 51 | Neisseria Meningitidis |
| 11 | Cutibacterium acnes | 52 | Prevotella salivae |
| 12 | Prevotella buccae | 53 | Streptococcus massiliensis |
| 13 | Lactobacillus vaginalis | 54 | Aggregatibacter aphrophilus |
| 14 | Prevotella enoeca | 55 | Streptococcus agalactiae |
| 15 | Prevotella fusca | 56 | Prevotella maculosa |
| 16 | Haemophilus paraphrohaemolyticus | 57 | Prevotella oris |
| 17 | Lactobacillus oris | 58 | Solobacterium moorei |
| 18 | Alloprevotella rava | 59 | Streptococcus infantis |
| 19 | Prevotella marshii | 60 | Veillonella atypica |
| 20 | Neisseria gonorrhoeae | 61 | Selenomonas infelix |
| 21 | Haemophilus pittmaniae | 62 | Streptococcus australis |
| 22 | Haemophilus sputorum | 63 | Streptococcus sinensis |
| 23 | Prevotella pleuritidis | 64 | Alloprevotella tannerae |
| 24 | Prevotella oralis | 65 | Streptococcus intermedius |
| 25 | Granulicatella elegans | 66 | Bacteroidetes oral taxon 274 |
| 26 | Streptococcus peroris | 67 | Actinomyces timonensis |
| 27 | Streptococcus pseudopneumoniae | 68 | Streptococcus anginosus |
| 28 | Johnsonella ignava | 69 | Selenomonas flueggei |
| 29 | Prevotella scopos | 70 | Streptococcus parasanguinis |
| 30 | Actinomyces graevenitzii | 71 | Leptotrichia hofstadii |
| 31 | Campylobacter curvus | 72 | Leptotrichia wadei |
| 32 | Neisseria lactamica | 73 | Atopobium rimae |
| 33 | Actinomyces slackii | 74 | Campylobacter gracilis |
| 34 | Haemophilus influenzae | 75 | Aggregatibacter segnis |
| 35 | Neisseria polysaccharea | 76 | Stomatobaculum longum |
| 36 | Haemophilus haemolyticus | 77 | Candidate division TM7 single-cell isolate TM7a |
| 37 | Oribacterium sinus | 78 | Neisseria bacilliformis |
| 38 | Eubacterium infirmum | 79 | Neisseria subflava |
| 39 | Gemella sanguinis | 80 | Kingella denitrificans |
| 40 | Streptococcus salivarius | 81 | Lachnoanaerobaculum saburreum |
| 41 | Prevotella micans | 82 | Prevotella oulorum |

# FIG. 12 (contd.)

## Commensal Flora (contd.)

| 83 | Porphyromonas catoniae |
| 84 | Capnocytophaga sputigena |
| 85 | Gemella morbillorum |
| 86 | Neisseria mucosa |
| 87 | Neisseria macacae |
| 88 | Actinomyces georgiae |
| 89 | Candidatus saccharibacteria oral taxon TM7x |
| 90 | Neisseria flavescens |
| 91 | Capnocytophaga granulosa |
| 92 | Streptococcus mitis |
| 93 | Selenomonas artemidis |
| 94 | Capnocytophaga ochracea |
| 95 | Morococcus cerebrosus |
| 96 | Kingella oralis |
| 97 | Neisseria sicca |
| 98 | Neisseria elongata |
| 99 | Granulicatella adiacens |
| 100 | Capnocytophaga gingivalis |
| 101 | Haemophilus parainfluenzae |
| 102 | Abiotrophia defectiva |
| 103 | Streptococcus oralis |
| 104 | Streptococcus cristatus |
| 105 | Streptococcus gordonii |
| 106 | Streptococcus sanguinis |
| 107 | Rothia aeria |
| 108 | Corynebacterium durum |
| 109 | Lautropia mirabilis |
| 110 | Actinomyces johnsonii |
| 111 | Actinomyces gerencseriae |
| 112 | Veillonella parvula |
| 113 | Actinomyces massiliensis |
| 114 | Candidate division TM7 single-cell isolate TM7c |
| 115 | Corynebacterium matruchotii |
| 116 | Actinomyces naeslundii |
| 117 | Actinomyces oris |
| 118 | Actinomyces viscosus |
| 119 | Candidate division TM7 single-cell isolate TM7b |

## Caries

| 1 | Acidipropionibacterium acidipropionici |
| 2 | Lactobacillus rhamnosus |
| 3 | Lactobacillus gasseri |
| 4 | Lactobacillus casei |
| 5 | Lactobacillus fermentum |
| 6 | Streptococcus sobrinus |
| 7 | Lactobacillus salivarius |
| 8 | Parascardovia denticolens |
| 9 | Prevotella histicola |
| 10 | Bifidobacterium dentium |
| 11 | Prevotella nigrescens |
| 12 | Rothia mucilaginosa |
| 13 | Propionibacterium acidifaciens |
| 14 | Scardovia wiggsiae |
| 15 | Streptococcus mutans |
| 16 | Rothia dentocariosa |

## Malodor

| 1 | Eubacterium saphenum |
| 2 | Eubacterium sulci |
| 3 | Eubacterium yurii |
| 4 | Prevotella shahii |
| 5 | Prevotella intermedia |
| 6 | Fusobacterium periodonticum |
| 7 | Centipeda periodontii |
| 8 | Prevotella pallens |
| 9 | Prevotella veroralis |
| 10 | Megasphaera micronuciformis |
| 11 | Candidate division sr1 bacterium MGEHA |
| 12 | Prevotella melaninogenica |
| 13 | Atopobium parvulum |
| 14 | Actinomyces odontolyticus |
| 15 | Veillonella dispar |

# FIG. 12 (contd.)

| Gingivitis / Periodontitis / Pathogenic | | Gingivitis / Periodontitis / Pathogenic (contd.) | |
|---|---|---|---|
| 1 | *Mycoplasma salivarium* | 33 | *Tannerella forsythia* |
| 2 | *Oribacterium asaccharolyticum* | 34 | *Treponema maltophilum* |
| 3 | *Leptotrichia goodfellowii* | 35 | *Porphyromonas gingivalis* |
| 4 | *Leptotrichia shahii* | 36 | *Parvimonas micra* |
| 5 | *Gemella haemolysans* | 37 | *Olsenella uli* |
| 6 | *Leptotrichia buccalis* | 38 | *Campylobacter rectus* |
| 7 | *Cardiobacterium valvarum* | 39 | *Streptococcus constellatus* |
| 8 | *Eikenella corrodens* | 40 | *Fusobacterium hwasookii* |
| 9 | *Actinomyces israelii* | 41 | *Slackia exigua* |
| 10 | *Pyramidobacter piscolens* | 42 | *Porphyromonas endodontalis* |
| 11 | *Treponema putidum* | 43 | *Treponema socranskii* |
| 12 | *Hallella seregens* | 44 | *Cryptobacterium curtum* |
| 13 | *Prevotella multisaccharivorax* | 45 | *Anaeroglobus geminatus* |
| 14 | *Pseudoramibacter alactolyticus* | 46 | *Campylobacter showae* |
| 15 | *Eggerthia catenaformis* | 47 | *Peptostreptococcus stomatis* |
| 16 | *Treponema denticola* | 48 | *Fusobacterium nucleatum* |
| 17 | *Filifactor alocis* | 49 | *Eubacterium brachy* |
| 18 | *Prevotella baroniae* | 50 | *Prevotella denticola* |
| 19 | *Peptoanaerobacter stomatis* | 51 | *Selenomonas sputigena* |
| 20 | *Mitsuokella sp. Oral taxon 131* | 52 | *Prevotella loescheii* |
| 21 | *Prevotella bivia* | 53 | *Dialister invisus* |
| 22 | *Eubacterium nodatum* | 54 | *Selenomonas noxia* |
| 23 | *Treponema lecithinolyticum* | 55 | *Cardiobacterium hominis* |
| 24 | *Olsenella profusa* | 56 | *Pseudopropionibacterium propionicum* |
| 25 | *Prevotella aurantiaca* | 57 | *Actinomyces dentalis* |
| 26 | *Fretibacterium fastidiosum* | 58 | *Actinobaculum sp. Oral taxon 183* |
| 27 | *Treponema medium* | 59 | *Actinomyces cardiffensis* |
| 28 | *Treponema vincentii* | 60 | *Scardovia inopinata* |
| 29 | *Mogibacterium timidum* | 61 | *Bulleidia extructa* |
| 30 | *Mogibacterium sp. CM50* | 62 | *Streptococcus pyogenes* |
| 31 | *Aggregatibacter actinomycetemcomitans* | 63 | *Catonella morbi* |
| 32 | *Shuttleworthia satelles* | | |

FIG. 13

Legend: +2 log, +1 log, No Change, -1 log, -2 log, -3 log

FIG. 13 (contd.)

Commensal Flora

## FIG. 13 (contd.)

| Commensal Flora | | | Commensal Flora (contd.) |
|---|---|---|---|
| 1 | Bacteroides thetaiotaomicron | 42 | Leptotrichia trevisanii |
| 2 | Desulfobulbus sp. oral taxon 041 | 43 | Neisseria cinerea |
| 3 | Prevotella dentalis | 44 | Prevotella nanceiensis |
| 4 | Lactobacillus paracasei | 45 | Prevotella multiformis |
| 5 | Chloroflexi bacterium oral taxon 439 | 46 | Campylobacter concisus |
| 6 | Dialister micraerophilus | 47 | Prevotella saccharolytica |
| 7 | Bacteroides uniformis | 48 | Streptococcus vestibularis |
| 8 | Alloscardovia omnicolens | 49 | Streptococcus pneumoniae |
| 9 | Oribacterium parvum | 50 | Peptoniphilus lacrimalis |
| 10 | Haemophilus parahaemolyticus | 51 | Neisseria Meningitidis |
| 11 | Cutibacterium acnes | 52 | Prevotella salivae |
| 12 | Prevotella buccae | 53 | Streptococcus massiliensis |
| 13 | Lactobacillus vaginalis | 54 | Aggregatibacter aphrophilus |
| 14 | Prevotella enoeca | 55 | Streptococcus agalactiae |
| 15 | Prevotella fusca | 56 | Prevotella maculosa |
| 16 | Haemophilus paraphrohaemolyticus | 57 | Prevotella oris |
| 17 | Lactobacillus oris | 58 | Solobacterium moorei |
| 18 | Alloprevotella rava | 59 | Streptococcus infantis |
| 19 | Prevotella marshii | 60 | Veillonella atypica |
| 20 | Neisseria gonorrhoeae | 61 | Selenomonas infelix |
| 21 | Haemophilus pittmaniae | 62 | Streptococcus australis |
| 22 | Haemophilus sputorum | 63 | Streptococcus sinensis |
| 23 | Prevotella pleuritidis | 64 | Alloprevotella tannerae |
| 24 | Prevotella oralis | 65 | Streptococcus intermedius |
| 25 | Granulicatella elegans | 66 | Bacteroidetes oral taxon 274 |
| 26 | Streptococcus peroris | 67 | Actinomyces timonensis |
| 27 | Streptococcus pseudopneumoniae | 68 | Streptococcus anginosus |
| 28 | Johnsonella ignava | 69 | Selenomonas flueggei |
| 29 | Prevotella scopos | 70 | Streptococcus parasanguinis |
| 30 | Actinomyces graevenitzii | 71 | Leptotrichia hofstadii |
| 31 | Campylobacter curvus | 72 | Leptotrichia wadei |
| 32 | Neisseria lactamica | 73 | Atopobium rimae |
| 33 | Actinomyces slackii | 74 | Campylobacter gracilis |
| 34 | Haemophilus influenzae | 75 | Aggregatibacter segnis |
| 35 | Neisseria polysaccharea | 76 | Stomatobaculum longum |
| 36 | Haemophilus haemolyticus | 77 | Candidate division TM7 single-cell isolate TM7a |
| 37 | Oribacterium sinus | 78 | Neisseria bacilliformis |
| 38 | Eubacterium infirmum | 79 | Neisseria subflava |
| 39 | Gemella sanguinis | 80 | Kingella denitrificans |
| 40 | Streptococcus salivarius | 81 | Lachnoanaerobaculum saburreum |
| 41 | Prevotella micans | 82 | Prevotella oulorum |

# FIG. 13 (contd.)

## Commensal Flora (contd.)

| 83 | Porphyromonas catoniae |
|---|---|
| 84 | Capnocytophaga sputigena |
| 85 | Gemella morbillorum |
| 86 | Neisseria mucosa |
| 87 | Neisseria macacae |
| 88 | Actinomyces georgiae |
| 89 | Candidatus saccharibacteria oral taxon TM7x |
| 90 | Neisseria flavescens |
| 91 | Capnocytophaga granulosa |
| 92 | Streptococcus mitis |
| 93 | Selenomonas artemidis |
| 94 | Capnocytophaga ochracea |
| 95 | Morococcus cerebrosus |
| 96 | Kingella oralis |
| 97 | Neisseria sicca |
| 98 | Neisseria elongata |
| 99 | Granulicatella adiacens |
| 100 | Capnocytophaga gingivalis |
| 101 | Haemophilus parainfluenzae |
| 102 | Abiotrophia defectiva |
| 103 | Streptococcus oralis |
| 104 | Streptococcus cristatus |
| 105 | Streptococcus gordonii |
| 106 | Streptococcus sanguinis |
| 107 | Rothia aeria |
| 108 | Corynebacterium durum |
| 109 | Lautropia mirabilis |
| 110 | Actinomyces johnsonii |
| 111 | Actinomyces gerencseriae |
| 112 | Veillonella parvula |
| 113 | Actinomyces massiliensis |
| 114 | Candidate division TM7 single-cell isolate TM7c |
| 115 | Corynebacterium matruchotii |
| 116 | Actinomyces naeslundii |
| 117 | Actinomyces oris |
| 118 | Actinomyces viscosus |
| 119 | Candidate division TM7 single-cell isolate TM7b |

FIG. 14

FIG. 14 (contd.)

Gingivitis / Periodontitis / Pathogenic

# FIG. 14 (contd.)

## Caries

| 1 | Acidipropionibacterium acidipropionici |
|---|---|
| 2 | Lactobacillus rhamnosus |
| 3 | Lactobacillus gasseri |
| 4 | Lactobacillus casei |
| 5 | Lactobacillus fermentum |
| 6 | Streptococcus sobrinus |
| 7 | Lactobacillus salivarius |
| 8 | Parascardovia denticolens |
| 9 | Prevotella histicola |
| 10 | Bifidobacterium dentium |
| 11 | Prevotella nigrescens |
| 12 | Rothia mucilaginosa |
| 13 | Propionibacterium acidifaciens |
| 14 | Scardovia wiggsiae |
| 15 | Streptococcus mutans |
| 16 | Rothia dentocariosa |

## Malodor

| 1 | Eubacterium saphenum |
|---|---|
| 2 | Eubacterium sulci |
| 3 | Eubacterium yurii |
| 4 | Prevotella shahii |
| 5 | Prevotella intermedia |
| 6 | Fusobacterium periodonticum |
| 7 | Centipeda periodontii |
| 8 | Prevotella pallens |
| 9 | Prevotella veroralis |
| 10 | Megasphaera micronuciformis |
| 11 | Candidate division sr1 bacterium MGEHA |
| 12 | Prevotella melaninogenica |
| 13 | Atopobium parvulum |
| 14 | Actinomyces odontolyticus |
| 15 | Veillonella dispar |

# FIG. 14 (contd.)

| | Gingivitis / Periodontitis / Pathogenic | | Gingivitis / Periodontitis / Pathogenic (contd.) |
|---|---|---|---|
| 1 | Mycoplasma salivarium | 33 | Tannerella forsythia |
| 2 | Oribacterium asaccharolyticum | 34 | Treponema maltophilum |
| 3 | Leptotrichia goodfellowii | 35 | Porphyromonas gingivalis |
| 4 | Leptotrichia shahii | 36 | Parvimonas micra |
| 5 | Gemella haemolysans | 37 | Olsenella uli |
| 6 | Leptotrichia buccalis | 38 | Campylobacter rectus |
| 7 | Cardiobacterium valvarum | 39 | Streptococcus constellatus |
| 8 | Eikenella corrodens | 40 | Fusobacterium hwasookii |
| 9 | Actinomyces israelii | 41 | Slackia exigua |
| 10 | Pyramidobacter piscolens | 42 | Porphyromonas endodontalis |
| 11 | Treponema putidum | 43 | Treponema socranskii |
| 12 | Hallella seregens | 44 | Cryptobacterium curtum |
| 13 | Prevotella multisaccharivorax | 45 | Anaeroglobus geminatus |
| 14 | Pseudoramibacter alactolyticus | 46 | Campylobacter showae |
| 15 | Eggerthia catenaformis | 47 | Peptostreptococcus stomatis |
| 16 | Treponema denticola | 48 | Fusobacterium nucleatum |
| 17 | Filifactor alocis | 49 | Eubacterium brachy |
| 18 | Prevotella baroniae | 50 | Prevotella denticola |
| 19 | Peptoanaerobacter stomatis | 51 | Selenomonas sputigena |
| 20 | Mitsuokella sp. Oral taxon 131 | 52 | Prevotella loescheii |
| 21 | Prevotella bivia | 53 | Dialister invisus |
| 22 | Eubacterium nodatum | 54 | Selenomonas noxia |
| 23 | Treponema lecithinolyticum | 55 | Cardiobacterium hominis |
| 24 | Olsenella profusa | 56 | Pseudopropionibacterium propionicum |
| 25 | Prevotella aurantiaca | 57 | Actinomyces dentalis |
| 26 | Fretibacterium fastidiosum | 58 | Actinobaculum sp. Oral taxon 183 |
| 27 | Treponema medium | 59 | Actinomyces cardiffensis |
| 28 | Treponema vincentii | 60 | Scardovia inopinata |
| 29 | Mogibacterium timidum | 61 | Bulleidia extructa |
| 30 | Mogibacterium sp. CM50 | 62 | Streptococcus pyogenes |
| 31 | Aggregatibacter actinomycetemcomitans | 63 | Catonella morbi |
| 32 | Shuttleworthia satelles | | |

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 955 232 A1 (BORK PEER [DE]) 16 December 2015 (2015-12-16) * abstract * * paragraphs [0001], [0024], [0025], [0028], [0052], [0053], [0057] * | 1-14 | INV. C12Q1/6809 |
| X | JACOBT NEARING ET AL: "Identifying biases and their potential solutions in human microbiome studies", MICROBIOME, BIOMED CENTRAL LTD, LONDON, UK, vol. 9, no. 1, 18 May 2021 (2021-05-18), pages 1-22, XP021291018, DOI: 10.1186/S40168-021-01059-0 | 6,7 | |
| Y | * abstract; figure 1 * * page 12 – page 13 * * page 14, column 1, paragraph 2 * * page 7, column 1, paragraph 2 * | 1,8,10 | |
| Y | ANDRZEJ TKACZ ET AL: "Absolute quantitation of microbiota abundance in environmental samples", MICROBIOME, BIOMED CENTRAL LTD, LONDON, UK, vol. 6, no. 1, 19 June 2018 (2018-06-19), pages 1-13, XP021257642, DOI: 10.1186/S40168-018-0491-7 * abstract * * page 14, column 2, paragraph 2 * | 1,8,10 | TECHNICAL FIELDS SEARCHED (IPC)  C12Q |

-/--

The present search report has been drawn up for all claims

| Place of search | Date of completion of the search | Examiner |
|---|---|---|
| Munich | 20 February 2023 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 22 19 9525

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SMETS WENKE ET AL: "A method for simultaneous measurement of soil bacterial abundances and community composition via 16S rRNA gene sequencing", SOIL BIOLOGY AND BIOCHEMISTRY, PERGAMON, OXFORD, GB, vol. 96, 26 February 2016 (2016-02-26), pages 145-151, XP029493698, ISSN: 0038-0717, DOI: 10.1016/J.SOILBIO.2016.02.003 * the whole document * ----- | 1,8,10 | |
| Y | FRANK STAMMLER ET AL: "Adjusting microbiome profiles for differences in microbial load by spike-in bacteria", MICROBIOME, vol. 4, no. 1, 21 June 2016 (2016-06-21), XP055652333, DOI: 10.1186/s40168-016-0175-0 * abstract * * page 2, column 1, paragraph 4 * ----- | 1,8,10 | |
| A | GALLOWAY-PEÑA JESSICA ET AL: "Tools for Analysis of the Microbiome", DIGESTIVE DISEASES AND SCIENCES, SPRINGER NEW YORK LLC, US, vol. 65, no. 3, 31 January 2020 (2020-01-31), pages 674-685, XP037041059, ISSN: 0163-2116, DOI: 10.1007/S10620-020-06091-Y [retrieved on 2020-01-31] * abstract * ----- -/-- | 1-14 | TECHNICAL FIELDS SEARCHED (IPC) |

The present search report has been drawn up for all claims

| Place of search | Date of completion of the search | Examiner |
|---|---|---|
| Munich | 20 February 2023 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 9525

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | POUSSIN CARINE ET AL: "Interrogating the microbiome: experimental and computational considerations in support of study reproducibility", DRUG DISCOVERY TODAY, ELSEVIER, AMSTERDAM, NL, vol. 23, no. 9, 8 June 2018 (2018-06-08), pages 1644-1657, XP085471066, ISSN: 1359-6446, DOI: 10.1016/J.DRUDIS.2018.06.005 * abstract * | 1-14 | |
| A | JO JIHOON ET AL: "Microbial community analysis using high-throughput sequencing technology: a beginner's guide for microbiologists", THE JOURNAL OF MICROBIOLOGY, THE MICROBIOLOGICAL SOCIETY OF KOREA // HAN-GUG MISAENGMUL HAG-HOE, KR, vol. 58, no. 3, 27 February 2020 (2020-02-27), pages 176-192, XP037044464, ISSN: 1225-8873, DOI: 10.1007/S12275-020-9525-5 [retrieved on 2020-02-27] * abstract * | 1-14 | |

TECHNICAL FIELDS
SEARCHED    (IPC)

The present search report has been drawn up for all claims

| Place of search | Date of completion of the search | Examiner |
|---|---|---|
| Munich | 20 February 2023 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 22 19 9525

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2023

| Patent document cited in search report | | Publication date | Patent family member(s) | | Publication date |
|---|---|---|---|---|---|
| EP 2955232 | A1 | 16-12-2015 | DK | 2955232 T3 | 27-11-2017 |
| | | | EP | 2955232 A1 | 16-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63340634
- US 63252818
- US 4683195 A
- US 4965 A
- US 188 A
- US 4683202 A
- US 4800159 A
- US 5210015 A
- US 6174670 B
- US 5399491 A
- US 6287824 B
- US 5854033 A
- US 20060024711 A
- US 20130059737 A1, Drmanac
- US 20180135120 A1, Ruan
- US 20180330044 A1, Lawley

**Non-patent literature cited in the description**

- **FOUHY, F. ; CLOONEY, A.G. ; STANTON, C. et al.** 16S rRNA gene sequencing of mock microbial populations- impact of DNA extraction method, primer choice and sequencing platform. *BMC Microbiol,* 2016, vol. 16, 123
- **JIAN C. et al.** Quantitative PCR provides a simple and accessible method for quantitative microbiota profiling. *PLoS ONE,* 2020, vol. 15 (1
- **CHEN C ; KHALEEL SS ; HUANG H ; WU CH.** Software for pre-processing Illumina next-generation sequencing short read sequences. *Source Code Biol Med,* 2014